# EUROPEAN PATENT SPECIFICATION

(11) **EP 2 198 030 B1**
(45) Date of publication and mention of the grant of the patent: **14.09.2016**
(21) Application number: 08837113.3
(22) Date of filing: 10.10.2008
(51) Int. Cl.: C07K 14/325, C12N 15/82

(54) **SYNTHETIC GENES ENCODING CRY1AC**
FÜR CRY1AC KODIERENDE SYNTHETISCHE GENE
GÈNES SYNTHÉTIQUES CODANT POUR CRY1AC

(30) Priority: 10.10.2007 US 978970 P
(43) Date of publication of application: 23.06.2010
(60) Divisional application: 13183867.4
(73) Proprietor: Athenix Corporation, Research Triangle Park, NC 27709 (US)
(72) Inventor: CAROZZI, Nadine, Raleigh, NC 27615 (US); DESAI, Nalini, Chapel Hill, NC 27514 (US); TOMSO, Daniel, J., Bahama, NC 27503 (US)
(74) Representative: Desaix, Anne
(86) International application number: PCT/US2008/079478
(87) International publication number: WO 2009/049126

(56) References cited:
- EP-A- 0 359 472
- EP-A- 0 385 962
- WO-A1-2009/023639
- US-A- 5 380 831
- US-A- 6 075 185
- US-A1- 2001 003 849
- US-A1- 2003 046 726
- US-A1- 2003 192 078
- ADAMCZYK JOHN J JR ET AL: "Potential factors impacting season-long expression of Cry1Ac in 13 commercial varieties of Bollgard(R) cotton" BIOSIS,, 1 January 1900 (1900-01-01), XP002372218
- DONG H. Z. ET AL: "Variability od Endotoxin Expression in Bt Transgenic Cotton" J.AGRONOMY & CROP SCIENCE, vol. 193, 2007, pages 21-29, XP002512063

## Description

### FIELD OF THE INVENTION

This invention relates to the field of molecular biology. Provided are novel nucleotide sequences that encode pesticidal proteins. These proteins and the nucleic acid sequences that encode them are useful in preparing pesticidal formulations and in the production of transgenic pest-resistant plants.

### BACKGROUND OF THE INVENTION

*Bacillus thuringiensis* is a Gram-positive spore forming soil bacterium characterized by its ability to produce crystalline inclusions that are specifically toxic to certain orders and species of insects, but are harmless to plants and other non-targeted organisms. For this reason, compositions including *Bacillus thuringiensis* strains or their insecticidal proteins can be used as environmentally-acceptable insecticides to control agricultural insect pests or insect vectors for a variety of human or animal diseases.

The use of commercial, transgenic crops expressing *Bacillus thuringiensis* (Bt) toxins has escalated in recent years because of their advantages over traditional chemical insecticides. However, native *cry* genes have poor coding capacity in plants (Murray et al. (1991) PlantMol. Biol. 16, 1035-1050). A number of strategies have been devised to increase the expression of *Bt* genes. These include the use of *Arabidopsis thaliana* small subunit leader and transit peptide to increase transcription and translation efficiency *(*Wong et al. (1992) Plant Mol. Biol. 20, 81-93), the combination of the 35S promoter and the castor bean intron (Fujimoto et al. (1993) Bio/Technology 11:1151-1155), and amplification of the toxin gene in chloroplasts (McBride et al. (1995) Bio/Technology 13, 362-365) as well as modification of codon usage to match codon preference in plants (Fujimoto *et al.* (1993); Wünn, et al. (1996) Biotechnology 14:171-176; Nayak et al.. (1997) Proc. Natl. Acad. Sci. USA 94:2111-2116; Sardana, et al. (1996) Plant Cell Rep. 15, 677-681; Perlak et al. (1990) Bio/Technology 8, 939-943; Perlak et al. (1991) Proc. Natl. Acad. Sci. USA 88, 3324-3328).

US2003/046726 A1 provides synthetic sequences of *cryIA(b)* genes. The examples clearly test the effectiveness of these genes on corn borers, sugarcane borers and European corn borers. US2003/046726 A1 does not even generally describe that their synthetic sequences of *cryIA(b)* genes can be used to manage both heliothine pests such as *Helicoverpa zea* and Lepidopteran pests such as the European corn borer.

Since commercialization in 1996, transgenic cotton plants containing a modified form of the *cryIAc* gene from the soil bacterium, *Bacillus thuringlensis* Berliner, (Bt) (Bollgard®, Monsanto Co., St. Louis, MO) have been used extensively to manage lepidopteran pests. However, some heliothines are not adequately controlled with this technology (Bacheler and Mott (1997) In: Dugger P, Richter D, eds. Beltwide Cotton Conference Proceedings, pp. 858-861.Memphis: National Cotton Council; Smith (1998) In: Dugger P, Richter D, eds, Beltwide Cotton Conference Proceedings, pp. 965-966.Memphis: National Cotton Council), and the difference has been attributed to varying levels of CrylAc.

### SUMMARY OF INVENTION

Compositions and methods for conferring pesticidal activity to bacteria, plants, plant cells, tissues and seeds are provided. Compositions include synthetic nucleic acid molecules encoding CrylAc pesticidal and insectidal polypeptides, vectors comprising those nucleic acid molecules, and host cells comprising the vectors. The nucleotide sequences can be used in DNA constructs or expression cassettes for transformation and expression in organisms, including microorganisms and plants. The synthetic nucleotide sequences are designed for expression in an organism including, but not limited to, a microorganism or a plant. Compositions also comprise transformed bacteria, plants, plant cells, tissues, and seeds.

In particular, isolated synthetic nucleic acid molecules are provided that encode a CrylAc pesticidal protein. In particular, the present invention provides for an isolated nucleic acid molecule comprising the nucleotide sequence set forth in SEQ ID NO: 1, 2, 3, 4, or 5. Nucleotide sequences that are complimentary to a nucleotide sequence are described herein or that hybridize to a sequence of the invention are described herein.

Methods are provided for producing the polypeptides of the invention, and for using those polypeptides for controlling or killing a lepidopteran pest. Methods and kits for detecting the nucleic acids and polypeptides of the invention in a sample are also included.

The compositions and methods of the invention are useful for the production of organisms with enhanced pest resistance or tolerance by improving the expression level of the CrylAc protein in the organism. These organisms and compositions comprising the organisms are desirable for agricultural purposes. The compositions of the invention are also useful for generating altered or improved proteins that have pesticidal activity, or for detecting the presence of pesticidal proteins or nucleic acids in products or organisms.

### DETAILED DESCRIPTION

The present invention is drawn to compositions and methods for regulating pest resistance or tolerance in organisms, particularly plants or plant cells. By "resistance" is intended that the pest (e.g., insect) is killed upon ingestion or other contact with the polypeptides of the invention. By "tolerance" is intended an impairment or reduction in the movement, feeding, reproduction, or other functions of the pest.

The methods involve transforming organisms with a synthetic nucleotide sequence encoding a CrylAc pesticidal protein of the invention. Several reports discuss problems with expression and/or toxicity of native *cry1Ac* as well as various modified versions encoding Cryl Ac. See for example, Barton et al. (1987) Plant Physiol. 85;1103-1109, United States Patent Publication No. 20010003849, and United States Patent No. 6,121,014. Provided herein are nucleotide sequences useful for preparing plants and microorganisms that possess pesticidal activity. Thus, transformed bacteria, plants, plant cells, plant tissues and seeds are provided. The sequences find use in the construction of expression vectors for subsequent transformation into organisms of interest, as probes for the isolation of other homologous (or partially homologous) genes, and for the generation of altered pesticidal proteins by methods known in the art, such as domain swapping or DNA shuffling. The proteins find use in controlling or killing lepidopteran pest populations and for producing compositions with pesticidal activity.

### Isolated Nucleic Acid Molecules

One aspect as described herein pertains to synthetic or recombinant nucleic acid molecules comprising nucleotide sequences encoding CrylAc proteins and polypeptides, as well as nucleic acid molecules sufficient for use as hybridization probes to identify nucleic acid molecules encoding proteins with regions of sequence homology. As used herein, the term "nucleic acid molecule" is intended to include DNA molecules (e.g., recombinant DNA, cDNA or genomic DNA) and RNA molecules (e.g., mRNA) and analogs of the DNA or RNA generated using nucleotide analogs. The nucleic acid molecule can be single-stranded or double-stranded, but preferably is double-stranded DNA.

Nucleotide sequences encoding the proteins of the present invention include the sequence set forth in SEQ ID NO: land complements thereof are disclosed herein. By "complement" is intended a nucleotide sequence that is sufficiently complementary to a given nucleotide sequence such that it can hybridize to the given nucleotide sequence to thereby form a stable duplex. The corresponding amino acid sequence for the pesticidal protein encoded by these nucleotide sequences is set forth in SEQ ID NO:6.

Nucleic acid molecules that are fragments of these nucleotide sequences encoding CrylAc pesticidal proteins are also described herein. By "fragment" is intended a portion of the nucleotide sequence encoding a pesticidal protein. A fragment of a nucleotide sequence may encode a biologically active portion of a pesticidal protein, or it may be a fragment that can be used as a hybridization probe or PCR primer using methods disclosed below. Nucleic acid molecules that are fragments of a nucleotide sequence encoding a pesticidal protein comprise at least about 50,100, 200, 300,400,500, 600, 700, 800,900, 1000, 1100,1200, 1300,1350,1400 contiguous nucleotides, or up to the number of nucleotides present in a full-length nucleotide sequence encoding the CrylAc protein disclosed herein, depending upon the intended use. By "contiguous" nucleotides is intended nucleotide residues that are immediately adjacent to one another. Fragments of the nucleotide sequences as disclosed herein will encode protein fragments that retain the biological activity of the pesticidal protein and, hence, retain pesticidal activity. By "retains activity" is intended that the fragment will encode a protein that has at least about 30%, at least about 50%, at least about 70%, 80%, 90%, 95% or higher of the pesticidal activity of the pesticidal protein. In one embodiment, the pesticidal activity is Lepidopteran activity. Methods for measuring pesticidal activity are well known in the art. See, for example, Czapla and Lang (1990) J. Econ. Entomol. 83:2480-2485; Andrews et al. (1988) Biochem. J. 252:199-206; Marrone et al. (1985) J. of Economic Entomology 78:290-293; and U.S. Patent No. 5,743,477.

A fragment of a nucleotide sequence encoding a Cry1Ac pesticidal protein that encodes a biologically active portion of a protein as disclosed herein will encode at least about 15, 25, 30, 50, 75,100, 125,150,175, 200,250, 300, 350, 400, 450 contiguous amino acids, or up to the total number of amino acids present in a full-length CrylA protein.

In various embodiments, the synthetic *crylAc* nucleotide sequences described herein result in the production of a a level of CrylAc in an organism that is at least about 5% higher, at least about 10% higher, at least about 20%, at least about 30%, at least about 40%, at least about 50% higher, at least about 60%, at least about 70%, at least about 80%, at least about 90% higher, at least about 100%, at least about 150%, at least about 200%, or higher, or at least about 3-fold, al least about 4-fold, at least about 5-fold, at least about 6-fold, at least about 7-fold, at least about 8-fold, at least about 9-fold, at least about 10-fold, or higher, than the level of CrylAc protein produced in an organism expressing the native *crylAc* gene (GENBANK Accession No. BTU87793; SEQ ID NO:8).

Preferred pesticidal proteins as described herein are encoded by a nucleotide sequence sufficiently identical to the nucleotide sequence of SEQ ID NO:1. By "sufficiently identical" is intended an amino acid or nucleotide sequence that has at least about 60% or 65% sequence identity, about 70% or 75% sequence identity, about 80% or 85% sequence identity, about 90%, 91%, 92%, 93%, 94%, 95%, 96%, 97%, 98%, 99% or greater sequence identity compared to a reference sequence using one of the alignment programs described herein using standard parameters. One of skill in the art will recognize that these values can be appropriately adjusted to determine corresponding identity of proteins encoded by two nucleotide sequences by taking into account codon degeneracy, amino acid similarity, reading frame positioning, and the like.

To determine the percent identity of two amino acid sequences or of two nucleic acids, the sequences are aligned for optimal comparison purposes. The percent identity between the two sequences is a function of the number of identical positions shared by the sequences (i.e., percent identity = number of identical positions/total number of positions (e.g., overlapping positions) x 100). In one embodiment, the two sequences are the same length. The percent identity between two sequences can be determined using techniques similar to those described below, with or without allowing gaps. In calculating percent identity, typically exact matches are counted.

The determination of percent identity between two sequences can be accomplished using a mathematical algorithm. A non limiting example of a mathematical algorithm utilized for the comparison of two sequences is the algorithm of Karlin and Altschul (1990) Proc. Natl, Acad. Sci. USA 87:2264, modified as in Karlin and Altschul (1993) Proc. Natl. Acad. Sci. USA 90:5873-5877. Such an algorithm is incorporated into the BLAST and BLASTX programs of Altschul et al. (1990) J. MoL Biol. 215:403. BLAST nucleotide searches can be performed with the BLASTN program, score = 100, wordlength = 12, to obtain nucleotide sequences homologous to pesticidal-like nucleic acid molecules of the invention. BLAST protein searches can be performed with the
BLASTX program, score = 50, wordlength = 3, to obtain amino acid sequences homologous to pesticidal protein molecules of the invention. To obtain gapped alignments for comparison purposes, Gapped BLAST (in BLAST 2.0) can be utilized as described in Altschul et al. (1997) Nucleic Acids Res. 25:3389. Alternatively, PSI-Blast can be used to perform an iterated search that detects distant relationships between molecules. See *Altschul et al.* (1997) *supra*. When utilizing BLAST, Gapped BLAST, and PSI-Blast programs, the default parameters of the respective programs (e.g., BLASTX and BLASTN) can be used. Alignment may also be performed manually by inspection.

Another non-limiting example of a mathematical algorithm utilized for the comparison of sequences is the ClustalW algorithm (Higgins et al. (1994) Nucleic Acids Res. 22:4673-4680). ClustalW compares sequences and aligns the entirety of the amino acid or DNA sequence, and thus can provide data about the sequence conservation of the entire amino acid sequence. The ClustalW algorithm is used in several commercially available DNA/amino acid analysis software packages, such as the ALIGNX module of the Vector NTI Program Suite (Invitrogen Corporation, Carlsbad, CA). After alignment of amino acid sequences with ClustalW, the percent amino acid identity can be assessed. A non-limiting example of a software program useful for analysis of ClustalW alignments is GENEDOC™. GENEDOC™ (Karl Nicholas) allows assessment of amino acid (or DNA) similarity and identity between multiple proteins. Another non-limiting example of a mathematical algorithm utilized for the comparison of sequences is the algorithm of Myers and Miller (1988) CABIOS 4:11-17. Such an algorithm is incorporated into the ALIGN program (version 2.0), which is part of the GCG Wisconsin Genetics Software Package, Version 10 (available from Accelrys, Inc., 9685 Scranton Rd., San Diego, CA, USA). When utilizing the ALIGN program for comparing amino acid sequences, a PAM120 weight residue table, a gap length penalty of 12, and a gap penalty of 4 can be used.

Unless otherwise stated, GAP Version 10, which uses the algorithm of Needleman and Wunsch (1970) J. Mol. Biol. 48(3):443-453, will be used to determine sequence identity or similarity using the following parameters: % identity and % similarity for a nucleotide sequence using GAP Weight of 50 and Length Weight of 3, and the nwsgapdna.cmp scoring matrix; % identity or % similarity for an amino acid sequence using GAP weight of 8 and length weight of 2, and the BLOSUM62 scoring program. Equivalent programs may also be used. By "equivalent program" is intended any sequence comparison program that, for any two sequences in question, generates an alignment having identical nucleotide residue matches and an identical percent sequence identity when compared to the corresponding alignment generated by GAP Version 10.

Variant nucleic acid molecules are also described herein. "Variants" of the pesticidal protein encoding nucleotide sequences include those sequences that encode the pesticidal proteins disclosed herein but that differ conservatively because of the degeneracy of the genetic code as well as those that are sufficiently identical as discussed above. Naturally occurring allelic variants can be identified with the use of well-known molecular biology techniques, such as polymerase chain reaction (PCR) and hybridization techniques as outlined bellow. Variant nucleotide sequences also include synthetically derived nucleotide sequences that have been generated, for example, by using site-directed mutagenesis but which still encode the pesticidal proteins disclosed in the present invention as discussed below. Variant proteins are biologically active, that is they continue to possess the desired biological activity of the native protein, that is, pesticidal activity. By "retains activity" is intended that the variant will have at least about 30%, at least about 50%, at least about 70%, or at least about 80% of the pesticidal activity of the native CrylAc protein. Methods for measuring pesticidal activity are well known in the art. See, for example, Czapla and Lang (1990) J. Econ. Entomol. 83: 2480-2485; Andrews et al. (1988) Biochem J.252:199-206; Marrone et al. (1985) J. of Economic Entomology 78:290-293; and U.S. Patent No. 5,74,3,477.

The skilled artisan will further appreciate that changes can be introduced by mutation of the nucleotide sequences of the invention thereby leading to changes in the amino acid sequence of the encoded pesticidal proteins, without altering the biological activity of the proteins. Thus, variant isolated nucleic acid molecules can be created by introducing one or more nucleotide substitutions, additions, or deletions into the corresponding nucleotide sequence disclosed herein, such that one or more amino acid substitutions, additions or deletions are introduced into the encoded protein. Mutations can be introduced by standard techniques, such as site-directed mutagenesis and PCR-mediated mutagenesis. Such variant nucleotide sequences are also encompassed by the present invention,

For example, conservative amino acid substitutions may be made at one or more, predicted, nonessential amino acid residues. A '"nonessential" amino acid residue is a residue that can be altered from the wild-type sequence of a pesticidal protein without altering the biological activity, whereas an "essential" amino acid residue is required for biological activity. A "conservative amino acid substitution" is one in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine).

Delta-endotoxins generally have five conserved sequence domains, and three conserved structural domains (see, for example, de Maagd et al. (2001) Trends Genetics 17:193-199). The first conserved structural domain consists of seven alpha helices and is involved in membrane insertion and pore formation. Domain II consists of three beta-sheets arranged in a Greek key configuration, and domain III consists of two antiparallel beta-sheets in "jelly-roll" formation (de Maagd *et al.,* 2001, supra). Domains II and III are involved in receptor recognition and binding, and are therefore considered determinants of toxin specificity.

Amino acid substitutions may be made in nonconserved regions that retain function. In general, such substitutions would not be made for conserved amino acid residues, or for amino acid residues residing within a conserved motif, where such residues are essential for protein activity. Examples of residues that are conserved and that may be essential for protein activity include, for example, residues that are identical between all proteins contained in an alignment of similar or related toxins to the sequences of the invention. Examples of residues that are conserved but that may allow conservative amino acid substitutions and still retain activity include, for example, residues that have only conservative substitutions between all proteins contained in an alignment of similar or related toxins to the sequences of the invention. However, one of skill in the art would understand that functional variants may have minor conserved or nonconserved alterations in the conserved residues.

Alternatively, variant nucleotide sequences can be made by introducing mutations randomly along all or part of the coding sequence, such as by saturation mutagenesis, and the resultant mutants can be screened for ability to confer pesticidal activity to identify mutants that retain activity. Following mutagenesis, the encoded protein can be expressed recombinantly, and the activity of the protein can be determined using standard assay techniques.

Using methods such as PCR, hybridization, and the like corresponding pesticidal sequences can be identified, such sequences having substantial identity to the synthetic *crylAc* sequences of the invention. See, for example, Sambrook and Russell (2001) Molecular Cloning: A Laboratory Manual. (Cold Spring Harbor Laboratory Press, Cold Spring Harbor, NY) and Innis, et al. (1990) PCR Protocols: A Guide to Methods and Applications (Academic Press, NY).

In a hybridization method, all or part of the synthetic *cryIAc* nucleotide sequence can be used to screen cDNA or genomic libraries. Methods for construction of such cDNA and genomic libraries are generally known in the art and are disclosed in Sambrook and Russell, 2001, *supra*. The so-called hybridization probes may be genomic DNA fragments, cDNA fragments, RNA fragments, or other oligonucleotides, and may be labeled with a detectable group such as ³²P, or any other detectable marker, such as other radioisotopes, a fluorescent compound, an enzyme, or an enzyme co-factor. Probes for hybridization can be made by labeling synthetic oligonucleotides based on the known synthetic *cry1Ac* nucleotide sequences disclosed herein. Degenerate primers designed on the basis of conserved nucleotides or amino acid residues in the nucleotide sequence or encoded amino acid sequence can additionally be used. The probe typically comprises a region of nucleotide sequence that hybridizes under stringent conditions to at least about 12, at least about 25, at least about 50, 75, 100, 125, 150, 175, or 200 consecutive nucleotides of the synthetic *cry1Ac* nucleotide sequences of the invention or a fragment or variant thereof. Methods for the preparation of probes for hybridization are generally known in the art and are disclosed in Sambrook and Russell, 2001, *supra*.

For example, an entire pesticidal protein sequence disclosed herein, or one or more portions thereof, may be used as a probe capable of specifically hybridizing to corresponding pesticidal protein-like sequences and messenger RNAs. To achieve specific hybridization under a variety of conditions, such probes include sequences that are unique and are preferably at least about 10 nucleotides in length, or at least about 20 nucleotides in length. Such probes may be used to amplify corresponding pesticidal sequences from a chosen organism by PCR. This technique may be used to isolate additional coding sequences from a desired organism or as a diagnostic assay to determine the presence of coding sequences in an organism. Hybridization techniques include hybridization screening of plated DNA libraries (either plaques or colonies; see, for example, Sambrook et al. (1989) Molecular Cloning: A Laboratory Manual (2d ed., Cold Spring Harbor Laboratory Press, Cold Spring Harbor, New York).

Hybridization of such sequences may be carried out under stringent conditions. By "stringent conditions" or "stringent hybridization conditions" is intended conditions under which a probe will hybridize to its target sequence to a detectably greater degree than to other sequences (e.g., at least 2-fold over background). Stringent conditions are sequence-dependent and will be different in different circumstances. By controlling the stringency of the hybridization and/or washing conditions, target sequences that are 100% complementary to the probe can be identified (homologous probing). Alternatively, stringency conditions can be adjusted to allow some mismatching in sequences so that lower degrees of similarity are detected (heterologous probing). Generally, a probe is less than about 1000 nucleotides in length, preferably less than 500 nucleotides in length.

Typically, stringent conditions will be those in which the salt concentration is less than about 1.5 M Na ion, typically about 0.01 to 1.0 M Na ion concentration (or other salts) at pH 7.0 to 8.3 and the temperature is at least about 30°C for short probes (e.g., 10 to 50 nucleotides) and at least about 60°C for long probes (e.g., greater than 50 nucleotides). Stringent conditions may also be achieved with the addition of destabilizing agents such as formamide. Exemplary low stringency conditions include hybridization with a buffer solution of 30 to 35% formamide, 1 M NaCl, 1% SDS (sodium dodecyl sulphate) at 37°C, and a wash in 1X to 2X SSC (20X SSC = 3.0 M NaCl/0.3 M trisodium citrate) at 50 to 55°C. Exemplary moderate stringency conditions include hybridization in 40 to 45% formamide, 1.0 M NaCl, 1% SDS at 37°C, and a wash in 0.5X to 1X SSC at 55 to 60°C. Exemplary high stringency conditions include hybridization in 50% formamide, 1 M NaCl, 1% SDS at 37°C, and a wash in 0.1X SSC at 60 to 65°C. Optionally, wash buffers may comprise about 0.1% to about 1% SDS. Duration of hybridization is generally less than about 24 hours, usually about 4 to about 12 hours.

Specificity is typically the function of post-hybridization washes, the critical factors being the ionic strength and temperature of the final wash solution. For DNA-DNA hybrids, the Tₘ can be approximated from the equation of Meinkoth and Wahl (1984) Anal. Biochem. 138:267-284: Tₘ = 81.5°C + 16.6 (log M) + 0.41 (%GC) - 0.61 (% form) - 500/L; where M is the molarity of monovalent cations, %GC is the percentage of guanosine and cytosine nucleotides in the DNA, % form is the percentage of formamide in the hybridization solution, and L is the length of the hybrid in base pairs. The Tₘ is the temperature (under defined ionic strength and pH) at which 50% of a complementary target sequence hybridizes to a perfectly matched probe. Tₘ is reduced by about 1°C for each 1 % of mismatching; thus, Tₘ, hybridization, and/or wash conditions can be adjusted to hybridize to sequences of the desired identity. For example, if sequences with ≥90% identity are sought, the Tₘ can be decreased 10°C. Generally, stringent conditions are selected to be about 5°C lower than the thermal melting point (Tₘ) for the specific sequence and its complement at a defined ionic strength and pH. However, severely stringent conditions can utilize a hybridization and/or wash at 1, 2, 3, or 4°C lower than the thermal melting point (Tₘ); moderately stringent conditions can utilize a hybridization and/or wash at 6, 7, 8, 9, or 10°C lower than the thermal melting point (Tₘ); low stringency conditions can utilize a hybridization and/or wash at 11, 12, 13, 14, 15, or 20°C lower than the thermal melting point (Tₘ). Using the equation, hybridization and wash compositions, and desired Tₘ, those of ordinary skill will understand that variations in the stringency of hybridization and/or wash solutions are inherently described. If the desired degree of mismatching results in a Tₘ of less than 45°C (aqueous solution) or 32°C (formamide solution), it is preferred to increase the SSC concentration so that a higher temperature can be used. An extensive guide to the hybridization of nucleic acids is found in Tijssen (1993) Laboratory Techniques in Biochemistry and Molecular Biology-Hybridization with Nucleic Acid Probes, Part I, Chapter 2 (Elsevier, New York); and Ausubel et ai, eds. (1995) Current Protocols in Molecular Biology, Chapter 2 (Greene Publishing and Wiley-Interscience, New York). See Sambrook et al. (1989) Molecular Cloning: A Laboratory Manual (2d ed., Cold Spring Harbor Laboratory Press, Cold Spring Harbor, New York).

### Isolated Proteins and Variants and Fragments Thereof

Variant CrylAc proteins are also described herein. By "variant CrylAc protein" is intended a biologically-active variant or fragment of the amino acid sequence set forth in SEQ ID NO:6. "Fragments" or "biologically active portions" include polypeptide fragments comprising amino acid sequences sufficiently identical to the amino acid sequence set forth in SEQ ID NO:6, and that exhibit pesticidal activity. A biologically active portion of a CrylAc protein can be a polypeptide that is, for example, 10, 25, 50, 100, 150, 200, 250 or more amino acids in length. Such biologically active portions can be prepared by recombinant techniques and evaluated for pesticidal activity. Methods for measuring pesticidal activity are well known in the art. See, for example, Czapla and Lang (1990) J. Econ. Entomol. 83:2480-2485; Andrews et al. (1988) Biochem. J. 252:199-206; Marrone et al. (1985) J. of Economic Entomology 78:290-293; and U.S. Patent No. 5,743,477. As used here, a fragment comprises at least 8 contiguous amino acids of SEQ ID NO:6. Other fragments, however, such as any fragment in the protein greater than about 10, 20, 30, 50, 100, 150, 200, 250, or 300 amino acids are described herein.

By "variants" is intended proteins or polypeptides having an amino acid sequence that is at least about 60%, 65%, about 70%, 75%, about 80%, 85%, about 90%, 91%, 92%, 93%, 94%, 95%, 96%, 97%, 98% or 99% identical to the amino acid sequence of NO:5. Variants also include polypeptides encoded by a nucleic acid molecule that hybridizes to the nucleic acid molecule of SEQ ID NO:1, 2, 3,4, or 5, or a complement thereof, under stringent conditions. Variants include polypeptides that differ in amino acid sequence due to mutagenesis. Variant proteins described herein are biologically active, that is they continue to possess the desired biological activity of the native protein, that is, retaining pesticidal activity. Methods for measuring pesticidal activity are well known in the art. See, for example, Czapla and Lang (1990) J. Econ. Entomol. 83:2480-2485; Andrews et al. (1988) Biochem. J. 252:199-206, Marrone et al. (1985) J. of Economic Entomology 78:290-293; and U.S. Patent No. 5,743,477.

Bacterial genes, such as the synthetic *cry1Ac* genes of this invention, quite often possess multiple methionine initiation codons in proximity to the start of the open reading frame. Often, translation initiation at one or more of these start cordons will lead to generation of a functional protein. These start codons can include ATG codons. However, bacteria such as *Bacillus sp.* also recognize the codon GTG as a start codon, and proteins that initiate translation at GTG codons contain a methionine at the first amino acid. Furthermore, it is not often determined *a priori* which of these codons are used naturally in the bacterium. Thus, it is understood that use of one of the alternate methionine codons in the synthetic nucleotide sequences disclosed herein may also lead to generation of pesticidal proteins.

Antibodies to the polypeptides as described herein, or to variants or fragments thereof, are described herein. Methods for producing antibodies are well known in the art (see, for example, Harlow and Lane (1988) Antibodies: A Laboratory Manual, Cold Spring Harbor Laboratory, Cold Spring Harbor, NY; U.S. Patent No. 4,196,265).

### Altered or Improved Variants

It is recognized that synthetic *crylAc* nucleotide sequences disclosed herein may be further altered by various methods, and that these alterations may resuit in DNA sequences encoding proteins with amino acid sequences different than the native CrylAc protein. This protein may be altered in various ways including amino acid substitutions, deletions, truncations, and insertions of one or more amino acids of NO:5, including up to about 2, about 3, about 4, about 5, about 6, about 7, about 8, about 9, about 10, about 15, about 20, about 25, about 30, about 35, about 40, about 45, about 50, about 55, about 60, about 65, about 70, about 75, about 80, about 85, about 90, about 100, about 105, about 110, about 115, about 120, about 125, about 130, about 135, about 140, about 145, about 150, about 155, or more amino acid substitutions, deletions or insertions. Methods for such manipulations are generally known in the art. For example, amino acid sequence variants of CrylAc can be prepared by mutations in the DNA. This may also be accomplished by one of several forms of mutagenesis and/or in directed evolution. In some aspects, the changes encoded in the amino acid sequence will not substantially affect the function of the protein. Such variants will possess the desired pesticidal activity. However, it is understood that the ability of a pesticidal protein to confer pesticidal activity may be improved by the use of such techniques upon the compositions as described herein. For example, one may express a pesticidal protein in host cells that exhibit high rates of base misincorporation during DNA replication, such as XL-1 Red (Stratagene, La Jolla, CA). After propagation in such strains, one can isolate the DNA (for example by preparing plasmid DNA, or by amplifying by PCR and cloning the resulting PCR fragment into a vector), culture the pesticidal protein mutations in a non-mutagenic strain, and identify mutated genes with pesticidal activity, for example by performing an assay to test for pesticidal activity. Generally, the protein is mixed and used in feeding assays. See, for example Marrone et al. (1985) J. of Economic Entomology 78:290-293. Such assays can include contacting plants with one or more pests and determining the plant's ability to survive and/or cause the death of the pests. Examples of mutations that result in increased toxicity are found in Schnepf et al. (1998) Microbiol. Mol. Biol. Rev. 62:775-806.

Alternatively, alterations may be made to the protein sequence of many proteins at the amino or carboxy terminus without substantially affecting activity. This can include insertions, deletions, or alterations introduced by modem molecular methods, such as PCR, including PCR amplifications that alter or extend the protein coding sequence by virtue of inclusion of amino acid encoding sequences in the oligonucleotides utilized in the
PCR amplification. Alternatively, the protein sequences added can include entire protein-coding sequences, such as those used commonly in the art to generate protein fusions. Such fusion proteins are often used to (1) increase expression of a protein of interest (2) introduce a binding domain, enzymatic activity, or epitope to facilitate either protein purification, protein detection, or other experimental uses known in the art (3) target secretion or translation of a protein to a subcellular organelle, such as the periplasmic space of Gram-negative bacteria, or the endoplasmic reticulum of eukaryotic cells, the latter of which often results in glycosylation of the protein.

Variant nucleotide and amino acid sequences as described herein also encompass sequences derived from mutagenic and recombinogenic procedures such as DNA shuffling. With such a procedure, one or more different pesticidal protein coding regions can be used to create a new pesticidal protein possessing the desired properties. In this manner, libraries of recombinant polynucleotides are generated from a population of related sequence polynucleotides comprising sequence regions that have substantial sequence identity and can be homologously recombined *in vitro* or *in vivo.* For example, using this approach, sequence motifs encoding a domain of interest may be shuffled between a synthetic *crylAc* sequence of the invention and other known pesticidal genes to obtain a new gene coding for a protein with an improved property of interest, such as an increased insecticidal activity. Strategies for such DNA shuffling are known in the art. See, for example, Stemmer (1994) Proc. Natl. Acad. Sci. USA 91:10747-10751 ; Stemmer (1994) Nature 370:389-391; Crameri et al. (1997) Nature Biotech. 15:436-438; Moore et al. (1997) J. Mol. Biol. 272:336-347; Zhang et al. (1997) Proc. Natl. Acad. Sci. USA 94:4504-4509; Crameri et al. (1998) Nature 391:288291; and U.S. Patent Nos. 5,605,793 and 5,837,458.

Domain swapping or shuffling is another mechanism for generating altered pesticidal proteins. Domains may be swapped between pesticidal proteins, resulting in hybrid or chimeric toxins with improved pesticidal activity or target spectrum. Methods for generating recombinant proteins and testing them for pesticidal activity are well known in the art (see, for example, Naimov et al. (2001) Appl. Environ. Microbiol. 67:5328-5330; de Maagd et al. (1996) Appl. Environ. Microbiol. 62:1537-1543; Ge et al. (1991) J. Biol. Chem. 266:17954-17958; Schnepf et al. (1990) J. Biol. Chem. 265:20923-20930; Rang et al. 1999) Appl. Environ. Microbiol. 65:2918-2925).

### Vectors

The pesticidal sequence of the invention may be provided in an expression cassette for expression in a plant of interest. By "plant expression cassette" is intended a DNA construct that is capable of resulting in the expression of a protein from an open reading frame in a plant cell. Typically these contain a promoter and a coding sequence. Often, such constructs will also contain a 3' untranslated region. Such constructs may contain a "signal sequence" or "leader sequence" to facilitate co-translational or post-translational transport of the peptide to certain intracellular structures such as the chloroplast (or other plastid), endoplasmic reticulum, or Golgi apparatus.

By "signal sequence" is intended a sequence that is known or suspected to result in cotranslational or post-translational peptide transport across the cell membrane. In eukaryotes, this typically involves secretion into the Golgi apparatus, with some resulting glycosylation. Insecticidal toxins of bacteria are often synthesized as protoxins, which are protolytically activated in the gut of the target pest (Chang (1987) Methods Enzymol. 153:507-516). In some embodiments of the present invention, the signal sequence is located in the native sequence, or may be derived from a sequence of the invention. By "leader sequence" is intended any sequence that when translated, results in an amino acid sequence sufficient to trigger co-translational transport of the peptide chain to a subcellular organelle. Thus, this includes leader sequences targeting transport and/or glycosylation by passage into the endoplasmic reticulum, passage to vacuoles, plastids including chloroplasts, mitochondria, and the like.

By "plant transformation vector" is intended a DNA molecule that is necessary for efficient transformation of a plant cell. Such a molecule may consist of one or more plant expression cassettes, and may be organized into more than one "vector" DNA molecule. For example, binary vectors are plant transformation vectors that utilize two non-contiguous DNA vectors to encode all requisite cis- and trans-acting functions for transformation of plant cells (Hellens and Mullineaux (2000) Trends in Plant Science 5:446-451). "Vector" refers to a nucleic acid construct designed for transfer between different host cells. "Expression vector" refers to a vector that has the ability to incorporate, integrate and express heterologous DNA sequences or fragments in a foreign cell. The cassette will include 5' and 3' regulatory sequences operably linked to a sequence of the invention. By "operably linked" is intended a functional linkage between a promoter and a second sequence, wherein the promoter sequence initiates and mediates transcription of the DNA sequence corresponding to the second sequence. Generally, operably linked means that the nucleic acid sequences being linked are contiguous and, where necessary to join two protein coding regions, contiguous and in the same reading frame. The cassette may additionally contain at least one additional gene to be cotransformed into the organism. Alternatively, the additional gene(s) can be provided on multiple expression cassettes.

"Promoter" refers to a nucleic acid sequence that functions to direct transcription of a downstream coding sequence. The promoter together with other transcriptional and translational regulatory nucleic acid sequences (also termed "control sequences") are necessary for the expression of a DNA sequence of interest.

Such an expression cassette is provided with a plurality of restriction sites for insertion of the pesticidal sequence to be under the transcriptional regulation of the regulatory regions.

The expression cassette will include in the 5'-3' direction of transcription, a transcriptional and translational initiation region (i.e., a promoter), a DNA sequence of the invention, and a translational and transcriptional termination region (i.e., termination region) functional in plants. The promoter may be native or analogous, or foreign or heterologous, to the plant host and/or to the DNA sequence of the invention. Additionally, the promoter may be the natural sequence or alternatively a synthetic sequence. Where the promoter is "native" or "homologous" to the plant host, it is intended that the promoter is found in the native plant into which the promoter is introduced. Where the promoter is "foreign" or "heterologous" to the DNA sequence of the invention, it is intended that the promoter is not the native or naturally occurring promoter for the operably linked DNA sequence of the invention.

The termination region may be native with the transcriptional initiation region, may be native with the operably linked DNA sequence of interest, may be native with the plant host, or may be derived from another source (i.e., foreign or heterologous to the promoter, the DNA sequence of interest, the plant host, or any combination thereof). Convenient termination regions are available from the Ti-plasmid of *A. tumefaciens,* such as the octopine synthase and nopaline synthase termination regions. See also Guerineau et al. (1991) Mol. Gen. Genet. 262:141-144; Proudfoot (1991) Cell 64:671-674; Sanfacon et al. (1991) Genes Dev. 5:141-149; Mogen et al. (1990) Plant Cell 2:1261-1272; Munroe et al. (1990) Gene 91:151-158; Ballas et al. (1989) Nucleic Acids Res. 17:7891-7903; and Joshi et al. (1987) Nucleic Acid Res. 15:9627-9639.

In one aspect of the invention, synthetic DNA sequences are designed for a given polypeptide, such as the synthetic *cry1Ac* DNA sequences described herein. Expression of the open reading frame of the synthetic DNA sequence in a cell results in production of the polypeptide of the invention. Synthetic DNA sequences can be useful to simply remove unwanted restriction endonuclease sites, to facilitate DNA cloning strategies, to alter or remove any potential codon bias, to alter or improve GC content, to remove or alter alternate reading frames, and/or to alter or remove intron/exon splice recognition sites, polyadenylation sites, Shine-Delgarno sequences, unwanted promoter elements and the like that may be present in a native DNA sequence. It is also possible that synthetic DNA sequences may be utilized to introduce other improvements to a DNA sequence, such as introduction of an intron sequence, creation of a DNA sequence that in expressed as a protein fusion to organelle targeting sequences, such as chloroplast transit peptides, apoplast/vacuolar targeting peptides, or peptide sequences that result in retention of the resulting peptide in the endoplasmic reticulum. Synthetic genes can also be synthesized using host cell-preferred codons for improved expression, or may be synthesized using codons at a host-preferred codon usage frequency. See, for example, Campbell and Gowri (1990) Plant Physiol. 92:1-11; U.S. Patent Nos. 6,320,100; 6,075,185; 5,380,831; and 5,436,391, U.S. Published Application Nos. 20040005600 and 20010003849, and Murray et al. (1989) Nucleic Acids Res. 17:477-498. In one embodiment, the pesticidal protein is targeted to the chloroplast for

In one embodiment, the pesticidal protein is targeted to the chloroplast for expression. In this manner, where the pesticidal protein is not directly inserted into the chloroplast, the expression cassette will additionally contain a nucleic acid encoding a transit peptide to direct the pesticidal protein to the chloroplasts. Such transit peptides are known in the art. See, for example, Von Heijne et al. (1991) PlantMol. Biol. Rep. 9:104-126; Clark et al. (1989) J. Biol. Chem. 264:17544-17550; Della-Cioppa et al. (1987) Plant Physiol. 84:965-968; Romer et al. (1993) Biochem. Biophys. Res. Commun. 196:1414-1421; and Shah et al. (1986) Science 233:478-481.

The pesticidal gene to be targeted to the chloroplast may be optimized for expression in the chloroplast to account for differences in codon usage between the plant nucleus and this organelle. In this manner, the nucleic acids of interest may be synthesized using chloroplast-preferred codons. See, for example, U.S. Patent No. 5,380,831.

### Plant Transformation

Methods of the invention involve introducing a nucleotide construct into a plant. By "introducing" is intended to present to the plant the nucleotide construct in such a manner that the construct gains access to the interior of a cell of the plant. The methods of the invention do not require that a particular method for introducing a nucleotide construct to a plant is used, only that the nucleotide construct gains access to the interior of at least one cell of the plant. Methods for introducing nucleotide constructs into plants are known in the art including, but not limited to, stable transformation methods, transient transformation methods, and virus-mediated methods.

By "plant" is intended whole plants, plant organs (e.g., leaves, stems, roots, etc.), seeds, plant cells, propagules, embryos and progeny of the same. Plant cells can be differentiated or undifferentiated (e.g. callus, suspension culture cells, protoplasts, leaf cells, root cells, phloem cells, pollen).

"Transgenic plants" or "transformed plants" or "stably transformed" plants or cells or tissues refers to plants that have incorporated or integrated exogenous nucleic acid sequences or DNA fragments into the plant cell. These nucleic acid sequences include those that are exogenous, or not present in the untransformed plant cell, as well as those that may be endogenous, or present in the untransformed plant cell. "Heterologous" generally refers to the nucleic acid sequences that are not endogenous to the cell or part of the native genome in which they are present, and have been added to the cell by infection, transfection, microinjection, electroporation, microprojection, or the like.

Transformation of plant cells can be accomplished by one of several techniques known in the art. The pesticidal gene of the invention may be modified to obtain or enhance expression in plant cells. Typically a construct that expresses such a protein would contain a promoter to drive transcription of the gene, as well as a 3' untranslated region to allow transcription termination and polyadenylation. The organization of such constructs is well known in the art. In some instances, it may be useful to engineer the gene such that the resulting peptide is secreted, or otherwise targeted within the plant cell. For example, the gene can be engineered to contain a signal peptide to facilitate transfer of the peptide to the endoplasmic reticulum. It may also be preferable to engineer the plant expression cassette to contain an intron, such that mRNA processing of the intron is required for expression.

Typically this "plant expression cassette" will be inserted into a "plant transformation vector". This plant transformation vector may be comprised of one or more DNA vectors needed for achieving plant transformation. For example, it is a common practice in the art to utilize plant transformation vectors that are comprised of more than one contiguous DNA segment. These vectors are often referred to in the art as "binary vectors". Binary vectors as well as vectors with helper plasmids are most often used for *Agrobacterium-*mediated transformation, where the size and complexity of DNA segments needed to achieve efficient transformation is quite large, and it is advantageous to separate functions onto separate DNA molecules. Binary vectors typically contain a plasmid vector that contains the cis-acting sequences required for T-DNA transfer (such as left border and right border), a selectable marker that is engineered to be capable of expression in a plant cell, and a "gene of interest" (a gene engineered to be capable of expression in a plant cell for which generation of transgenic plants is desired). Also present on this plasmid vector are sequences required for bacterial replication. The cis-acting sequences are arranged in a fashion to allow efficient transfer into plant cells and expression therein. For example, the selectable marker gene and the pesticidal gene are located between the left and right borders. Often a second plasmid vector contains the trans-acting factors that mediate T-DNA transfer from *Agrobacterium* to plant cells. This plasmid often contains the virulence functions (Vir genes) that allow infection of plant cells by *Agrobacterium,* and transfer of DNA by cleavage at border sequences and vir-mediated DNA transfer, as is understood in the art (Hellens and Mullineaux (2000) Trends in Plant Science 5:446-451). Several types *of Agrobacterium* strains (e.g. LBA4404, GV3101, EHA101, EHA105, etc.) can be used for plant transformation. The second plasmid vector is not necessary for transforming the plants by other methods such as microprojection, microinjection, electroporation, polyethylene glycol, etc.

In general, plant transformation methods involve transferring heterologous DNA into target plant cells (e.g. immature or mature embryos, suspension cultures, undifferentiated callus, protoplasts, etc.), followed by applying a maximum threshold level of appropriate selection (depending on the selectable marker gene) to recover the transformed plant cells from a group of untransformed cell mass. Explants are typically transferred to a fresh supply of the same medium and cultured routinely. Subsequently, the transformed cells are differentiated into shoots after placing on regeneration medium supplemented with a maximum threshold level of selecting agent. The shoots are then transferred to a selective rooting medium for recovering rooted shoot or plantlet. The transgenic plantlet then grows into a mature plant and produces fertile seeds (e.g. Hiei et al. (1994) The Plant Journal 6:271-282; Ishida et al. (1996) Nature Biotechnology 14:745-750). Explants are typically transferred to a fresh supply of the same medium and cultured routinely. A general description of the techniques and methods for generating transgenic plants are found in Ayres and Park (1994) Critical Reviews in Plant Science 13:219-239 and Bommineni and Jauhar (1997) Maydica 42:107-120. Since the transformed material contains many cells; both transformed and non-transformed cells are present in any piece of subjected target callus or tissue or group of cells. The ability to kill non-transformed cells and allow transformed cells to proliferate results in transformed plant cultures. Often, the ability to remove non-transformed cells is a limitation to rapid recovery of transformed plant cells and successful generation of transgenic plants.

Transformation protocols as well as protocols for introducing nucleotide sequences into plants may vary depending on the type of plant or plant cell, i.e., monocot or dicot, targeted for transformation. Generation of transgenic plants may be performed by one of several methods, including, but not limited to, microinjection, electroporation, direct gene transfer, introduction of heterologous DNA by *Agrobacterium* into plant cells (*Agrobacterium-*mediated transformation), bombardment of plant cells with heterologous foreign DNA adhered to particles, ballistic particle acceleration, aerosol beam transformation (U.S. Published Application No. 20010026941; U.S. Patent No. 4,945,050; International Publication No. WO 91/00915; U.S. Published Application No. 2002015066), Lec1 transformation, and various other non-particle direct-mediated methods to transfer DNA.

Methods for transformation of chloroplasts are known in the art. See, for example, Svab et al. (1990) Proc. Natl. Acad. Sci. USA 87:8526-8530; Svab and Maliga (1993) Proc. Natl. Acad. Sci. USA 90:913-917; Svab and Maliga (1993) EMBOJ. 12:601-606. The method relies on particle gun delivery of DNA containing a selectable marker and targeting of the DNA to the plastid genome through homologous recombination. Additionally, plastid transformation can be accomplished by transactivation of a silent plastid-borne transgene by tissue-preferred expression of a nuclear-encoded and plastid-directed RNA polymerase. Such a system has been reported in McBride et al. (1994) Proc. Natl. Acad. Sci. USA 91:7301-7305.

Following integration of heterologous foreign DNA into plant cells, one then applies a maximum threshold level of appropriate selection in the medium to kill the untransformed cells and separate and proliferate the putatively transformed cells that survive from this selection treatment by transferring regularly to a fresh medium. By continuous passage and challenge with appropriate selection, one identifies and proliferates the cells that are transformed with the plasmid vector. Molecular and biochemical methods can then be used to confirm the presence of the integrated heterologous gene of interest into the genome of the transgenic plant.

The cells that have been transformed may be grown into plants in accordance with conventional ways. See, for example, McCormick et al. (1986) Plant Cell Reports 5:81-84. These plants may then be grown, and either pollinated with the same transformed strain or different strains, and the resulting hybrid having constitutive expression of the desired phenotypic characteristic identified. Two or more generations may be grown to ensure that expression of the desired phenotypic characteristic is stably maintained and inherited and then seeds harvested to ensure expression of the desired phenotypic characteristic has been achieved. In this manner, the present invention provides transformed seed (also referred to as "transgenic seed") having a nucleotide construct of the invention, for example, an expression cassette of the invention, stably incorporated into their genome.

### Evaluation of Plant Transformation

Following introduction of heterologous foreign DNA into plant cells, the transformation or integration of heterologous gene in the plant genome is confirmed by various methods such as analysis of nucleic acids, proteins and metabolites associated with the integrated gene.

PCR analysis is a rapid method to screen transformed cells, tissue or shoots for the presence of incorporated gene at the earlier stage before transplanting into the soil (Sambrook and Russell (2001) Molecular Cloning: A Laboratory Manual. Cold Spring Harbor Laboratory Press, Cold Spring Harbor, NY). PCR is carried out using oligonucleotide primers specific to the gene of interest or *Agrobacterium* vector background, etc.

Plant transformation may be confirmed by Southern blot analysis of genomic DNA (Sambrook and Russell, 2001, *supra*). In general, total DNA is extracted from the transformant, digested with appropriate restriction enzymes, fractionated in an agarose gel and transferred to a nitrocellulose or nylon membrane. The membrane or "blot" is then probed with, for example, radiolabeled ³²P target DNA fragment to confirm the integration of introduced gene into the plant genome according to standard techniques (Sambrook and Russell, 2001, *supra*).

In Northern blot analysis, RNA is isolated from specific tissues of transformant, fractionated in a formaldehyde agarose gel, and blotted onto a nylon filter according to standard procedures that are routinely used in the art (Sambrook and Russell, 2001, *supra*). Expression of RNA encoded by the pesticidal gene is then tested by hybridizing the filter to a radioactive probe derived from a pesticidal gene, by methods known in the art (Sambrook and Russell, 2001, *supra*).

Western blot, biochemical assays and the like may be carried out on the transgenic plants to confirm the presence of protein encoded by the pesticidal gene by standard procedures (Sambrook and Russell, 2001, *supra*) using antibodies that bind to one or more epitopes present on the pesticidal protein.

### Pesticidal Activity in Plants

In another aspect of the invention, one may generate transgenic plants expressing a pesticidal protein that has pesticidal activity. Methods described above by way of example may be utilized to generate transgenic plants, but the manner in which the transgenic plant cells are generated is not critical to this invention. Methods known or described in the art such as *Agrobacterium*-mediated transformation, biolistic transformation, and non-particle-mediated methods may be used at the discretion of the experimenter. Plants expressing a pesticidal protein may be isolated by common methods described in the art, for example by transformation of callus, selection of transformed callus, and regeneration of fertile plants from such transgenic callus. In such process, one may use any gene as a selectable marker so long as its expression in plant cells confers ability to identify or select for transformed cells.

A number of markers have been developed for use with plant cells, such as resistance to chloramphenicol, the aminoglycoside G418, hygromycin, or the like. Other genes that encode a product involved in chloroplast metabolism may also be used as selectable markers. For example, genes that provide resistance to plant herbicides such as glyphosate, bromoxynil, or imidazolinone may find particular use. Such genes have been reported (Stalker et al. (1985) J. Biol. Chem. 263:6310-6314 (bromoxynil resistance nitrilase gene); and Sathasivan et al. (1990) Nucl. Acids Res. 18:2188 (AHAS imidazolinone resistance gene). Additionally, the genes disclosed herein are useful as markers to assess transformation of bacterial or plant cells. Methods for detecting the presence of a transgene in a plant, plant organ (e.g., leaves, stems, roots, etc.), seed, plant cell, propagule, embryo or progeny of the same are well known in the art. In one embodiment, the presence of the transgene is detected by testing for pesticidal activity.

Fertile plants expressing a pesticidal protein may be tested for pesticidal activity, and the plants showing optimal activity selected for further breeding. Methods are available in the art to assay for pest activity. Generally, the protein is mixed and used in feeding assays. See, for example Marrone et al. (1985) J. of Economic Entomology 78:290-293.

The present invention may be used for transformation of any plant species, including, but not limited to, monocots and dicots. Examples of plants of interest include, but are not limited to, corn (maize), sorghum, wheat, sunflower, tomato, crucifers, peppers, potato, cotton, rice, soybean, sugarbeet, sugarcane, tobacco, barley, and oilseed rape, *Brassica* sp., alfalfa, rye, millet, safflower, peanuts, sweet potato, cassava, coffee, coconut, pineapple, citrus trees, cocoa, tea, banana, avocado, fig, guava, mango, olive, papaya, cashew, macadamia, almond, oats, vegetables, ornamentals, and conifers.

Vegetables include, but are not limited to, tomatoes, lettuce, green beans, lima beans, peas, and members of the genus *Curcumis* such as cucumber, cantaloupe, and musk melon. Ornamentals include, but are not limited to, azalea, hydrangea, hibiscus, roses, tulips, daffodils, petunias, carnation, poinsettia, and chrysanthemum. Preferably, plants of the present invention are crop plants (for example, maize, sorghum, wheat, sunflower, tomato, crucifers, peppers, potato, cotton, rice, soybean, sugarbeet, sugarcane, tobacco, barley, oilseed rape., etc.).

"Pest" includes but is not limited to, insects, fungi, bacteria, nematodes, mites, ticks, and the like. Insect pests include insects selected from the orders *Coleoptera, Diptera, Hymenoptera*, *Lepidoptera, Mallophaga, Homoptera*, *Hemiptera*, *Orthroptera*, *Thysanoptera*, *Dermaptera*, *Isoptera, Anoplura, Siphonaptera, Trichoptera,* etc., particularly *Coleoptera, Lepidoptera,* and *Diptera.*

The order *Coleoptera* includes the suborders *Adephaga* and *Polyphaga.* Suborder *Adephaga* includes the superfamilies *Caraboidea* and *Gyrinoidea,* while suborder *Polyphaga* includes the superfamilies *Hydrophiloidea, Staphylinoidea, Cantharoidea, Cleroidea, Elateroidea, Dascilloidea, Dryopoidea, Byrrhoidea, Cucujoidea, Meloidea, Mordelloidea, Tenebrionoidea, Bostrichoidea, Scarabaeoidea, Cerambycoidea, Chrysomeloidea,* and *Curculionoidea.* Superfamily *Caraboidea* includes the families *Cicindelidae, Carabidae,* and *Dytiscidae.* Superfamily *Gyrinoidea* includes the family *Gyrinidae.* Superfamily *Hydrophiloidea* includes the family *Hydrophilidae.* Superfamily *Staphylinoidea* includes the families *Silphidae* and *Staphylinidae.* Superfamily *Cantharoidea* includes the families *Cantharidae* and *Lampyridae.* Superfamily *Cleroidea* includes the families *Cleridae* and *Dermestidae.* Superfamily *Elateroidea* includes the families *Elateridae* and *Buprestidae.* Superfamily *Cucujoidea* includes the family *Coccinellidae.* Superfamily *Meloidea* includes the family *Meloidae.* Superfamily *Tenebrionoidea* includes the family *Tenebrionidae.* Superfamily *Scarabaeoidea* includes the families *Passalidae* and *Scarabaeidae.* Superfamily *Cerambycoidea* includes the family *Cerambycidae.* Superfamily *Chrysomeloidea* includes the family *Chrysomelidae.* Superfamily *Curculionoidea* includes the families *Curculionidae* and *Scolytidae.*

The order *Diptera* includes the Suborders *Nematocera, Brachycera,* and *Cyclorrhapha.* Suborder *Nematocera* includes the families *Tipulidae, Psychodidae, Calicidae, Ceratopogonidae, Chironomidae, Simuliidae, Bibionidae,* and *Cecidomyiidae.* Suborder *Brachycera* includes the families *Stratiomyidae, Tabanidae, Therevidae, Asilidae, Mydidae, Bombyliidae,* and *Dolichopodidae.* Suborder *Cyclorrhapha* includes the Divisions *Aschiza* and *Aschiza.* Division *Aschiza* includes the families *Phoridae, Syrphidae,* and *Conopidae.* Division *Aschiza* includes the Sections *Acalyptratae* and *Calyptratae.* Section *Acalyptratae* includes the families *Otitidae, Tephritidae, Agromyzidae,* and *Drosophilidae.* Section *Calyptratae* includes the families *Hippoboscidae,* Oestridae, *Tachinidae, Anthomyiidae, Muscidae, Calliphoridae,* and *Sarcophagidae.*

The order *Lepidoptera* includes the families *Papilionidae, Pieridae, Lycaenidae, Nymphalidae, Danaidae, Satyridae, Hesperiidae, Sphingidae, Saturniidae, Geometridae, Arctiidae, Noctuidae, Lymantriidae, Sesiidae,* and *Tineidae.*

Insect pests of the invention for the major crops include: Maize: *Ostrinia nubilalis,* European corn borer; *Agrotis ipsilon,* black cutworm; *Helicoverpa zea,* corn earworm; *Spodoptera frugiperda,* fall armyworm; *Diatraea grandiosella,* southwestern corn borer; *Elasmopalpus lignosellus,* lesser cornstalk borer; *Diatraea saccharalis,* surgarcane borer; *Diabrotica virgifera,* western corn rootworm; *Diabrotica longicornis barberi,* northern corn rootworm; *Diabrotica undecimpunctata howardi,* southern corn rootworm; *Melanotus* spp., wireworms; *Cyclocephala borealis,* northern masked chafer (white grub); *Cyclocephala immaculata*, southern masked chafer (white grub); *Popillia japonica,* Japanese beetle; *Chaetocnema pulicaria,* corn flea beetle; *Sphenophorus maidis,* maize billbug; *Rhopalosiphum maidis,* corn leaf aphid; *Anuraphis maidiradicis*, corn root aphid; *Blissus leucopterus leucopterus,* chinch bug; *Melanoplus femurrubrum,* redlegged grasshopper; *Melanoplus sanguinipes*, migratory grasshopper; *Hylemya platura,* seedcorn maggot; *Agromyza parvicornis,* corn blot leafminer; *Anaphothrips obscrurus,* grass thrips; *Solenopsis milesta*, thief ant; *Tetranychus urticae,* twospotted spider mite; Sorghum: *Chilo partellus,* sorghum borer; *Spodoptera frugiperda,* fall armyworm; *Helicoverpa zea,* corn earworm; *Elasmopalpus lignosellus,* lesser cornstalk borer; *Feltia subterranea,* granulate cutworm; *Phyllophaga crinita,* white grub; *Eleodes, Conoderus,* and *Aeolus* spp., wireworms; *Oulema melanopus,* cereal leaf beetle; *Chaetocnema pulicaria,* corn flea beetle; *Sphenophorus maidis,* maize billbug; *Rhopalosiphum maidis;* corn leaf aphid; *Sipha flava,* yellow sugarcane aphid; *Blissus leucopterus leucopterus,* chinch bug; *Contarinia sorghicola*, sorghum midge; *Tetranychus cinnabarinus,* carmine spider mite; *Tetranychus urticae,* twospotted spider mite; Wheat: *Pseudaletia unipunctata*, army worm; *Spodoptera frugiperda,* fall armyworm; *Elasmopalpus lignosellus,* lesser cornstalk borer; *Agrotis orthogonia,* western cutworm; *Elasmopalpus lignosellus,* lesser cornstalk borer; *Oulema melanopus,* cereal leaf beetle; *Hypera punctata,* clover leaf weevil; *Diabrotica undecimpunctata howardi,* southern corn rootworm; Russian wheat aphid; *Schizaphis graminum,* greenbug; *Macrosiphum avenae,* English grain aphid; *Melanoplus femurrubrum,* redlegged grasshopper; *Melanoplus differentialis*, differential grasshopper; *Melanoplus sanguinipes*, migratory grasshopper; *Mayetiola destructor,* Hessian fly; *Sitodiplosis mosellana,* wheat midge; *Meromyza americana,* wheat stem maggot; *Hylemya coarctata,* wheat bulb fly; *Frankliniella fusca*, tobacco thrips; *Cephus cinctus,* wheat stem sawfly; *Aceria tulipae,* wheat curl mite; Sunflower: *Suleima helianthana,* sunflower bud moth; *Homoeosoma electellum,* sunflower moth; *zygogramma exclamationis,* sunflower beetle; *Bothyrus gibbosus,* carrot beetle; *Neolasioptera murtfeldtiana,* sunflower seed midge; Cotton: *Heliothis virescens,* cotton budworm; *Helicoverpa zea,* cotton bollworm; *Spodoptera exigua,* beet armyworm; *Pectinophora gossypiella,* pink bollworm; *Anthonomus grandis*, boll weevil; *Aphis gossypii,* cotton aphid; *Pseudatomoscelis seriatus*, cotton fleahopper; *Trialeurodes abutilonea*, bandedwinged whitefly; *Lygus lineolaris,* tarnished plant bug; *Melanoplus femurrubrum,* redlegged grasshopper; *Melanoplus differentialis*, differential grasshopper; *Thrips tabaci,* onion thrips; *Franklinkiella fusca*, tobacco thrips; *Tetranychus cinnabarinus*, carmine spider mite; *Tetranychus urticae,* twospotted spider mite; Rice: *Diatraea saccharalis,* sugarcane borer; *Spodoptera frugiperda,* fall armyworm; *Helicoverpa zea,* corn earworm; *Colaspis brunnea,* grape colaspis; *Lissorhoptrus oryzophilus,* rice water weevil; *Sitophilus oryzae*, rice weevil; *Nephotettix nigropictus,* rice leafhopper; *Blissus leucopterus leucopterus,* chinch bug; *Acrosternum hilare*, green stink bug; Soybean: *Pseudoplusia includens,* soybean looper; *Anticarsia gemmatalis,* velvetbean caterpillar; *Plathypena scabra,* green cloverworm; *Ostrinia nubilalis,* European corn borer; *Agrotis ipsilon,* black cutworm; *Spodoptera exigua,* beet armyworm; *Heliothis virescens,* cotton budworm; *Helicoverpa zea,* cotton bollworm; *Epilachna varivestis,* Mexican bean beetle; *Myzus persicae,* green peach aphid; *Empoasca fabae,* potato leafhopper; *Acrosternum hilare,* green stink bug; *Melanoplus femurrubrum,* redlegged grasshopper; *Melanoplus differentialis,* differential grasshopper; *Hylemya platura,* seedcorn maggot; *Sericothrips variabilis*, soybean thrips; *Thrips tabaci,* onion thrips; *Tetranychus turkestani,* strawberry spider mite; *Tetranychus urticae,* twospotted spider mite; Barley: *Ostrinia nubilalis,* European corn borer; *Agrotis ipsilon,* black cutworm; *Schizaphis graminum,* greenbug; *Blissus leucopterus leucopterus,* chinch bug; *Acrosternum hilare*, green stink bug; *Euschistus servus***,** brown stink bug; *Delia platura,* seedcorn maggot; *Mayetiola destructor,* Hessian fly; *Petrobia latens,* brown wheat mite; Oil Seed Rape: *Brevicoryne brassicae,* cabbage aphid; *Phyllotreta cruciferae***,** Flea beetle; *Mamestra configurata,* Bertha armyworm; *Plutella xylostella,* Diamond-back moth; *Delia* ssp., Root maggots.

Nematodes include parasitic nematodes such as root-knot, cyst, and lesion nematodes, including *Heterodera* spp., *Meloidogyne* spp., and *Globodera* spp.; particularly members of the cyst nematodes, including, but not limited to, *Heterodera glycines* (soybean cyst nematode); *Heterodera schachtii* (beet cyst nematode); *Heterodera avenae* (cereal cyst nematode); and *Globodera rostochiensis* and *Globodera pailida* (potato cyst nematodes). Lesion nematodes include *Pratylenchus* spp.

### Methods for increasing plant yield

Methods for increasing plant yield are provided. The methods comprise introducing into a plant or plant cell a polynucleotide comprising a synthetic *cry1Ac* nucleotide sequence disclosed herein. As defined herein, the "yield" of the plant refers to the quality and/or quantity of biomass produced by the plant. By "biomass" is intended any measured plant product. An increase in biomass production is any improvement in the yield of the measured plant product. Increasing plant yield has several commercial applications. For example, increasing plant leaf biomass may increase the yield of leafy vegetables for human or animal consumption. Additionally, increasing leaf biomass can be used to increase production of plant-derived pharmaceutical or industrial products. An increase in yield can comprise any statistically significant increase including, but not limited to, at least a 1% increase, at least a 3% increase, at least a 5% increase, at least a 10% increase, at least a 20% increase, at least a 30%, at least a 50%, at least a 70%, at least a 100% or a greater increase in yield compared to a plant not expressing the synthetic *cry1Ac* nucleotide sequence.

### EXPERIMENTAL

### Example 1. Synthetic nucleotide sequences encoding Cry1Ac

Synthetic nucleotide sequences encoding CrylAc (SEQ ID NO:6) were designed. These sequences are represented herein by SEQ ID NO:1 *(synFLCy1Ac),* SEQ ID NO:2 *(synCry1Ac-variant1),* SEQ ID NO:3 *(synCry1AcB),* SEQ ID NO:4 *(synCry1AcC),* and SEQ ID NO:5 *(optCry1Acv02).*

### Example 2. Expression of synthetic cry1Ac sequences in Bacillus

The synthetic *cry1Ac* sequence is amplified by PCR and cloned into the *Bacillus* expression vector pAX916 by methods well known in the art. The resulting clone is assayed for expression of Cry1Ac protein after transformation into cells of a *cry(-) Bacillus thuringiensis* strain. A *Bacillus* strain containing the *synCry1Ac* clone and expressing the Cry1Ac insecticidal protein is grown in, for example, CYS media (10 g/l Bacto-casitone; 3 g/l yeast extract; 6 g/l KH₂PO₄; 14 g/l K₂HPO₄; 0.5 mM MgSO₄; 0.05 mM MnCl₂; 0.05 mM FeSO₄) until sporulation is evident by microscopic examination. Samples are prepared, and analyzed by polyacrylamide gel electrophoresis (PAGE).

### Example 3. Assays for Pesticidal Activity

The synthetic *cry1Ac* nucleotide sequences of the invention can be tested for their ability to produce pesticidal proteins. The ability of a pesticidal protein to act as a pesticide upon a pest is often assessed in a number of ways. One way well known in the art is to perform a feeding assay. In such a feeding assay, one exposes the pest to a sample containing either compounds to be tested, or control samples. Often this is performed by placing the material to be tested, or a suitable dilution of such material, onto a material that the pest will ingest, such as an artificial diet. The material to be tested may be composed of a liquid, solid, or slurry. The material to be tested may be placed upon the surface and then allowed to dry. Alternatively, the material to be tested may be mixed with a molten artificial diet, then dispensed into the assay chamber. The assay chamber may be, for example, a cup, a dish, or a well of a microtiter plate.

Assays for sucking pests (for example aphids) may involve separating the test material from the insect by a partition, ideally a portion that can be pierced by the sucking mouth parts of the sucking insect, to allow ingestion of the test material. Often the test material is mixed with a feeding stimulant, such as sucrose, to promote ingestion of the test compound.

Other types of assays can include microinjection of the test material into the mouth, or gut of the pest, as well as development of transgenic plants, followed by test of the ability of the pest to feed upon the transgenic plant. Plant testing may involve isolation of the plant parts normally consumed, for example, small cages attached to a leaf, or isolation of entire plants in cages containing insects.

Other methods and approaches to assay pests are known in the art, and can be found, for example in Robertson and Preisler, eds. (1992) Pesticide bioassays with arthropods, CRC, Boca Ration, FL. Alternatively, assays are commonly described in the journals *Arthropod Management Tests* and *Journal of Economic Entomology* or by discussion with members of the Entomological Society of America (ESA).

### Examples 4. Sectoring of syncrylAc Genes for Plant Expression

The coding regions of the invention are connected with appropriate promoter and terminator sequences for expression in plants. Such sequences are well known in the art and may include the rice actin promoter or maize ubiquitin promoter for expression in monocots, the *Arabidopsis* UBQ3 promoter or CaMV 35S promoter for expression in dicots, and the nos or PinII terminators. Techniques for producing and confirming promoter - gene - terminator constructs also are well known in the art.

Synthetic DNA sequences are designed and generated as described herein. These synthetic sequences have altered nucleotide sequence relative to the parent sequence, but encode proteins that are essentially identical to the parent Cry1Ac protein (e.g., SEQ ID NO:1, 2, 3, 4, or 5).

In another aspect of the invention, modified versions of the synthetic genes are designed such that the resulting peptide is targeted to a plant organelle, such as the endoplasmic reticulum or the apoplast. Peptide sequences known to result in targeting of fusion proteins to plant organelles are known in the art. For example, the N-terminal region of the acid phosphatase gene from the White Lupin *Lupinus albus* (GENEBANK® ID GI:14276838, Miller et al. (2001) Plant Physiology 127: 594-606) is known in the art to result in endoplasmic reticulum targeting of heterologous proteins. If the resulting fusion protein also contains an endoplasmic reticulum retention sequence comprising the peptide N-terminus-lysine-aspartic acid-glutamic acid-leucine (i.e., the "KDEL" motif (SEQ ID NO:7)) at the C-terminus, the fusion protein will be targeted to the endoplasmic reticulum. If the fusion protein lacks an endoplasmic reticulum targeting sequence at the C-terminus, the protein will be targeted to the endoplasmic reticulum, but will ultimately be sequestered in the apoplast.

Thus, this gene encodes a fusion protein that contains the N-terminal thirty-one amino acids of the acid phosphatase gene from the White Lupin *Lupinus albus* (GENBANK® ID GI:14276838 , Miller *et al.,* 2001, *supra*) fused to the N-terminus of the Cry1Ac sequence, as well as the KDEL sequence at the C-terminus. Thus, the resulting protein is predicted to be targeted the plant endoplasmic reticulum upon expression in a plant cell.

The plant expression cassettes described above are combined with an appropriate plant selectable marker to aid in the selection of transformed cells and tissues, and ligated into plant transformation vectors. These may include binary vectors from *Agrobacterium-*mediated transformation or simple plasmid vectors for aerosol or biolistic transformation.

### Example 5. Vectoring of syncry1Ac genes for Plant Expression

The coding region DNA of the *syncry1Ac* genes of the invention are operably connected with appropriate promoter and terminator sequences for expression in plants. Such sequences are well known in the art and may include the rice actin promoter or maize ubiquitin promoter for expression in monocots, the *Arabidopsis* UBQ3 promoter or CaMV 35S promoter for expression in dicots, and the nos or PinII terminators. Techniques for producing and confirming promoter - gene - terminator constructs also are well known in the art.

The plant expression cassettes described above are combined with an appropriate plant selectable marker to aid in the selections of transformed cells and tissues, and ligated into plant transformation vectors. These may include binary vectors from *Agrobacterium-*mediated transformation or simple plasmid vectors for aerosol or biolistic transformation.

### Example 6. Transformation of Maize Cells with the pesticidal protein genes described herein

Maize ears are best collected 8-12 days after pollination. Embryos are isolated from the ears, and those embryos 0.8-1.5 mm in size are preferred for use in transformation. Embryos are plated scutellum side-up on a suitable incubation media, such as DN62A5S media (3.98 g/L N6 Salts; 1 mL/L (of 1000x Stock) N6 Vitamins; 800 mg/L L-Asparagine; 100 mg/L Myo-inositol; 1.4 g/L L-Proline; 100 mg/L Casamino acids; 50 g/L sucrose; 1 mL/L (of 1 mg/mL Stock) 2,4-D). However, media and salts other than DN62A5S are suitable and are known in the art. Embryos are incubated overnight at 25°C in the dark. However, it is not necessary *per se* to incubate the embryos overnight.

The resulting explants are transferred to mesh squares (30-40 per plate), transferred onto osmotic media for about 30-45 minutes, then transferred to a beaming plate (see, for example, PCT Publication No. WO/0138514 and U.S. Patent No. 5,240,842).

DNA constructs designed to the genes of the invention in plant cells are accelerated into plant tissue using an aerosol beam accelerator, using conditions essentially as described in PCT Publication No. WO/0138514. After beaming, embryos are incubated for about 30 min on osmotic media, and placed onto incubation media overnight at 25°C in the dark. To avoid unduly damaging beamed explants, they are incubated for at least 24 hours prior to transfer to recovery media. Embryos are then spread onto recovery period media, for about 5 days, 25°C in the dark, then transferred to a selection media. Explants are incubated in selection media for up to eight weeks, depending on the nature and characteristics of the particular selection utilized. After the selection period, the resulting callus is transferred to embryo maturation media, until the formation of mature somatic embryos is observed. The resulting mature somatic embryos are then placed under low light, and the process of regeneration is initiated by methods known in the art. The resulting shoots are allowed to root on rooting media, and the resulting plants are transferred to nursery pots and propagated as transgenic plants.

### Materials

**DN62A5S Media**

| Components | Per Liter | Source |
|---|---|---|
| Chu's N6 Basal Salt Mixture (Prod. No. C 416) | 3.98 g/L | Phytotechnology Labs |
| Chu's N6 Vitamin Solution (Prod. No. C 149) | 1 mL/L (of 1000x Stock) | Phytotechnology Labs |
| L-Asparagine | 800 mg/L | Phytotechnology Labs |
| Myo-inositol | 100 mg/L | Sigma |
| L-Proline | 1.4 g/L | Phytotechnology Labs |
| Casamino acids | 100 mg/L | Fisher Scientific |
| Sucrose | 50 g/L | Phytotechnology Labs |
| 2,4-D (Prod. No. D-7299) | 1 mL/L (of 1 mg/mL Stock) | Sigma |

The pH of the solution is adjusted to pH 5.8 with 1N KOH/1N KCl, Gelrite (Sigma) is added at a concentration up to 3g/L, and the media is autoclaved. After cooling to 50°C, 2 ml/L of a 5 mg/ml stock solution of silver nitrate (Phytotechnology Labs) is added.

### Example 7. Transformation of the syncry1Ac genes of the invention in Plant Cells by Agrobacterium-Mediated Transformation

Ears are best collected 8-12 days after pollination. Embryos are isolated from the ears, and those embryos 0.8-1.5 mm in size are preferred for use in transformation. Embryos are plated scutellum side-up on a suitable incubation media, and incubated overnight at 25°C in the dark. However, it is not necessary *per se* to incubate the embryos overnight. Embryos are contacted with an *Agrobacterium* strain containing the appropriate vectors for Ti plasmid mediated transfer for about 5-10 min, and then plated onto co-cultivation media for about 3 days (25°C in the dark). After co-cultivation, explants are transferred to recovery period media for about five days (at 25°C in the dark). Explants are incubated in selection media for up to eight weeks, depending on the nature and characteristics of the particular selection utilized. After the selection period, the resulting callus is transferred to embryo maturation media, until the formation of mature somatic embryos is observed. The resulting mature somatic embryos are then placed under low light, and the process of regeneration is initiated as known in the art.

### Example 8. Pesticidal activity against Lepidopteran pests

Transgenic corn events containing constructs developed to reduce damage caused by Lepidopteran insect pests were evaluated in a series of field trials to determine their level of resistance to the European corn borer *(Ostrinia nubilalis* Hübner) and corn earworm *(Helicoverpa zea* Boddie). Leaf damage ratings and stalk tunneling measurements were used to evaluate events for resistance to ECB. An ear damage rating was used to quantify the amount of ear feeding damage by corn earworm.

T2 events generated from two constructs were evaluated in field trials. Construct A contain the *synCry1Ac*-*variant1* gene (SEQ ID NO:2) driven by the TripPro5 promoter (US Patent Publication No. 20060218662). Construct B contained the *synFLCry1Ac* gene (SEQ ID NO:1) driven by the TrpPro5B promoter (US Patent Publication No. 20060218662). Both Hi-II and a susceptible hybrid were used as negative controls. The seed was packeted and arranged into randomized block design experiments in an isolated field site in Iowa. The trials were planted in ground that was fallow the previous year.

Agronomic practices used were representative of those used for corn production in the U.S. Corn Belt except that no insecticides were used. The plot area was disked and harrowed prior to planting to establish the seed bed. Liquid fertilizer was applied prior to planting. Weeds were controlled by use of both a pre-emerge application of and a post-emerge application of commercial herbicides, as well as spot treatment to control late emerging weeds.
**European Corn Borer (ECB) Trial.** For the first generation evaluations, all plants in the ECB trials were infested with ECB larvae on two separate dates. Plants were infested into the whorl of each plant using a field infestor that was calibrated to deliver 25 larvae per dose. Each plant was infested with 2 doses on each infestation date for a total infestation of 100 larvae per plant. Plots were evaluated for 1^{st} generation damage using the Guthrie Leaf Rating Scale (Guthrie et al. (1960) Leaf and sheath feeding resistance to the European corn borer in eight inbred lines of dent corn. Ohio Agric. Res. Dev. Cent. Res. Bull. 860). Negative segregants were removed before rating of the the plots. All remaining plants were infested with ECB larvae for the second generation evaluation. Larvae were prepared as described for the 1^{st} generation evaluations. In total, 150 larvae were applied to each plant over the three dates. Plants were evaluated for second generation damage by splitting the plant longitudinally from 3 nodes above the primary ear to the ground. The amount of tunneling in inches was measured for each stalk. The primary ear shank was also split and all tunnels were measured and added to the total. Any tunnels extending into the cob were also measured.
**Corn Earworm Trial.** Plants were infested with 50 ECB larvae during the whorl stage to identify positive and negative plants in each row. Corn earworm flights were monitored with a pheromone trap. A significant flight of corn earworm occurred in the area of this trial from mid to late August when plants were attractive to female moths. Through subsequent evaluations of individual ears in the trial, it was determined that the natural infestation was sufficient for corn earworm evaluations. Ear damage was scored at the appropriate time using a 1-9 visual scale (described in Table 1). First, positive and negative plants were identified by first generation ECB leaf damage. Ratings were taken on both positive and negative plants within each row. An average rating for the positive plants within the row was then calculated.
**Results and Discussion.** The infestations of ECB larvae produced sufficient levels of damage during both the 1^{st} and 2^{nd} generation. The negative controls had severe leaf damage. For corn earworm, the damage was more variable due to the natural infestation, but the majority of the negative ears had at least minor kernel damage.
In summary, the tested events exhibited commercially viable levels of Lepidopteran resistance; and many showed no visable damage or tunneling. Furthermore, it is clear that activity against corn earworm is present conferred by the synthetic *cry1Ac* constructs expressed in the transgenic lines.

**Table 1. European corn borer and Corn earworm data from field trials**

| Construct | Line | Leaf Rating (1-9) | Inches of Tunneling per plant | Corn Earworm Ear Damage Rating (1-9)² |
|---|---|---|---|---|
| A | 1 | 1 | 0 | 1.1 |
| A | 2 | 1 | 0 | . |
| A | 3 | 1 | 0.09 | . |
| A | 4 | 1.5 | 0 | 1.1 |
| B | 1 | 1 | 0 | 1.3 |
| B | 2 | 1.5 | 0 | 1.0 |
| B | 3 | 1 | 0 | 1.0 |
| B | 4 | 1 | 0.07 | . |
| Hi-II | | 7 | 5.18 | 2.8 |
| Susc. Hybrid | | 5 | 4.0 | 2.6 |

| | | | | |
|---|---|---|---|---|
| ¹ Lines in red are selected for advancement ² CEW Rating Scale: 1 = no damage, 2 = slight damage to silks, husks, and ear tip but no kernel damage, 3 = 1 - 2 kernels damaged, 4 = 0.1 - 1.0 cm of kernels damaged, 5 = 1.1 - 2.0 cm damaged, 6 = 2.1 - 3.0 cm damaged, 7 = 3.1 - 4.0 cm damaged, 8 = 4.1 - 5 cm damaged, 9 = >5.1 cm damaged | | | | |

## Claims

1. A nucleic acid molecule comprising a nucleotide sequence of SEQ ID NO: 1.

2. The nucleic acid molecule of claim 1, wherein said nucleotide sequence is operably linked to a promoter capable of directing expression of the nucleotide sequence in a plant cell and a termination region functional in plants.

3. A vector comprising the nucleic acid molecule of claim 1 or 2.

4. The vector of claim 3, further comprising a nucleic acid molecule encoding a heterologous polypeptide.

5. A host cell that contains the vector of claim 3.

6. A bacterial host cell that contains the vector of claim 3.

7. A plant host cell that contains the nucleic acid molecule of claims 1 or 2.

8. The host cell of claim 7, wherein said plant cell is a cell of a plant selected from the group consisting of maize, sorghum, wheat, cabbage, sunflower, tomato, crucifers, peppers, potato, cotton, rice, soybean, sugarbeet, sugarcane, tobacco, barley, and oilseed rape.

9. A transgenic seed comprising the nucleic acid molecule of claim 1 or 2.

10. A plant having stably incorporated into its genome a DNA construct comprising a nucleotide sequence that encodes a protein having pesticidal activity, wherein said nucleotide sequence comprises SEQ ID NO:1.

11. The plant of claim 10, wherein said plant is a plant cell.

12. The plant of claim 10, wherein said plant is selected from the group consisting of maize, sorghum, wheat, cabbage, sunflower, tomato, crucifers, peppers, potato, cotton, rice, soybean, sugarbeet, sugarcane, tobacco, barley, and oilseed rape.

13. A method for protecting a plant from a pest, comprising providing a plant or cell thereof comprising a nucleotide sequence that encodes a pesticidal polypeptide, wherein said nucleotide sequence comprises SEQ ID NO :1 and growing said plant or cell thereof in a field containing said pest.

14. The method of claim 13, wherein said plant produces a pesticidal polypeptide having pesticidal activity against a lepidopteran pest.

## Patentansprüche

1. Nukleinsäuremolekül, das eine Nukleotidsequenz gemäß SEQ ID NO: 1 umfasst.

2. Nukleinsäuremolekül nach Anspruch 1, wobei die Nukleotidsequenz auf funktionsfähige Weise mit einem Promoter verknüpft ist, der die Expression der Nukleotidsequenz in einer Pflanzenzelle steuern kann, und einer Terminations-region, die in Pflanzen funktional ist.

3. Vektor umfassend das Nukleinsäuremolekül nach Anspruch 1 oder 2.

4. Vektor nach Anspruch 3, weiterhin umfassend ein Nukleinsäuremolekül, das ein heterologes Polypeptid kodiert.

5. Wirtszelle, die den Vektor gemäß Anspruch 3 enthält.

6. Bakterielle Wirtszelle, die den Vektor gemäß Anspruch 3 enthält.

7. Pflanzliche Wirtszelle, die das Nukleinsäuremolekül nach Anspruch 1 oder 2 enthält.

8. Wirtszelle nach Anspruch 7, wobei die Pflanzenzelle eine Zelle einer Pflanze, ausgewählt aus der Gruppe bestehend aus Mais, Hirse, Weizen, Kohl, Sonnenblume, Tomate, Kreuzblütlern, Paprika, Kartoffel, Baumwolle, Reis, Sojabohne, Zuckerrübe, Zuckerrohr, Tabak, Gerste und Raps ist.

9. Transgener Samen umfassend das Nukleinsäuremolekül nach Anspruch 1 oder 2.

10. Pflanze mit stabil in ihr Genom inkorporiertem DNA-Konstrukt, das eine Nukleotidsequenz umfasst, die ein Protein mit pestizider Aktivität kodiert, wobei die Nukleotidsequenz SEQ ID NO: 1 umfasst.

11. Pflanze nach Anspruch 10, wobei die Pflanze eine Pflanzenzelle ist.

12. Pflanze nach Anspruch 10, wobei die Pflanze ausgewählt ist aus der Gruppe bestehend aus Mais, Hirse, Weizen, Kohl, Sonnenblume, Tomate, Kreuzblütlern, Paprika, Kartoffel, Baumwolle, Reis, Sojabohne, Zuckerrübe, Zuckerrohr, Tabak, Gerste und Raps.

13. herfahren zum Schützen einer Pflanzenzelle gegen einen Schädling, umfassend das Bereitstellen einer Pflanze oder einer Zelle davon, die eine Nukleotidsequenz umfasst, die ein pestizides Polypeptid kodiert, wobei die Nukleotidsequenz SEQ ID NO: 1 umfasst, und Anpflanzen der Pflanze oder der Zelle davon in einem Feld, das den Schädling enthält.

14. Verfahren nach Anspruch 13, wobei die Pflanze ein pestizides Polypeptid mit pestizider Aktivität gegenüber einem Lepidopteren-Schädling erzeugt.

## Revendications

1. Molécule d'acide nucléique comprenant une séquence nucléotidique de SEQ ID NO: 1.

2. Molécule d'acide nucléique selon la revendication 1, dans laquelle ladite séquence nucléotidique est liée de manière fonctionnelle à un promoteur apte à diriger l'expression de la séquence nucléotidique dans une cellule végétale et une région de terminaison fonctionnelle dans les plantes.

3. Vecteur comprenant la molécule d'acide nucléique selon la revendication 1 ou 2.

4. Vecteur selon la revendication 3, comprenant en outre une molécule d'acide nucléique codant un polypeptide hétérologue.

5. Cellule hôte qui contient le vecteur selon la revendication 3.

6. Cellule hôte bactérienne qui contient le vecteur selon la revendication 3.

7. Cellule hôte végétale qui contient la molécule d'acide nucléique selon la revendication 1 ou 2.

8. Cellule hôte selon la revendication 7, dans laquelle ladite cellule végétale est une cellule d'une plante choisie dans le groupe constitué par le maïs, le sorgho, le blé, le chou, le tournesol, la tomate, les crucifères, les poivrons, la pomme de terre, le coton, le riz, le soja, la betterave à sucre, la canne à sucre, le tabac, l'orge et le colza.

9. Graine transgénique comprenant la molécule d'acide nucléique selon la revendication 1 ou 2.

10. Plante ayant incorporé de manière stable dans son génome une construction d'ADN comprenant une séquence nucléotidique qui code une protéine ayant une activité pesticide, dans laquelle ladite séquence nucléotidique comprend SEQ ID NO: 1.

11. Plante selon la revendication 10, dans laquelle ladite plante est une cellule végétale.

12. Plante selon la revendication 10, dans laquelle ladite plante est choisie dans le groupe constitué par le maïs, le sorgho, le blé, le chou, le tournesol, la tomate, les crucifères, les poivrons, la pomme de terre, le coton, le riz, le soja, la betterave à sucre, la canne à sucre, le tabac, l'orge et le colza.

13. Procédé de protection d'une plante contre un ravageur, comprenant la fourniture d'une plante ou d'une cellule de celle-ci comprenant une séquence nucléotidique qui code un polypeptide pesticide, dans laquelle ladite séquence nucléotidique comprend SEQ ID NO: 1 et la culture de ladite plante ou cellule de celle-ci dans un champ contenant ledit ravageur.

14. Procédé selon la revendication 13, dans lequel ladite plante produit un polypeptide pesticide ayant une activité pesticide contre un ravageur lépidoptère.


### SEQUENCE LISTING

<110> Nadine Carozzi Nalini Desai Daniel J. Tomso
<120> SYNTHETIC GENES ENCODING CRY1AC
<130> 45600/363739
<150> 60/978,970
   <151> 2007-10-10
<160> 8
<170> FastSEQ for Windows Version 4.0
<210> 1
   <211> 3537
   <212> DNA
   <213> Artificial Sequence
<220>
   <223> synthetic nucleotide sequence encoding Cry1Ac (synFLCry1Ac)
<400> 1
   atggataaca accccaacat caacgagtgc atcccctaca actgcctctc aaaccccgag 60
   gtcgaggtcc tcggcggtga gcgtatcgag accggttaca cccctatcga tatcagcctg 120
   agcctgaccc agttcctcct ctcggagttc gtccccggtg ctggtttcgt tctgggtttg 180
   gtcgatatca tctggggcat cttcggcccc tcgcagtggg atgctttcct ggtccagatc 240
   gagcagttga tcaaccagcg catcgaggag ttcgccagga accaggctat ctcacgtctg 300
   gagggtttgt cgaacctcta ccaaatctac gccgagagct tccgcgagtg ggaggctgat 360
   cctactaacc ccgctttgcg tgaggagatg cgcatccagt tcaacgatat gaactcggcc 420
   ctgaccaccg ccatccccct cttcgctgtc cagaactacc aggtcccctt gctctcagtc 480
   tacgtccagg ctgctaacct ccatctgagc gtcctgaggg atgtctctgt cttcggccag 540
   aggtggggtt tcgatgctgc tactatcaac agccgctaca acgatctcac ccgcctgatc 600
   ggcaactaca ccgattacgc cgtccgttgg tacaacaccg gcctggagcg tgtttggggt 660
   cctgattcac gtgattgggt ccgttacaac cagttccgca gggagctcac cttgaccgtc 720
   ttggatatcg tcgccctctt ccccaactac gatagccgcc gttaccccat ccgtaccgtt 780
   agccagctca ctagggaaat ctacaccaac cccgtcctgg agaacttcga tggctcgttc 840
   cgtggttcag ctcagggtat cgagaggagc atccgttcgc ctcatctgat ggatatcctg 900
   aactcgatca ccatctacac cgatgcccat cgcggctact actactggtc gggccatcag 960
   atcatggcct cgcccgtcgg cttctcaggt cctgagttca ccttcccctt gtacggcacc 1020
   atgggtaacg ccgctcctca gcagaggatc gttgctcagt tgggtcaggg tgtttacagg 1080
   accctgagct ctaccctcta caggaggcct ttcaacatcg gcatcaacaa ccagcagctg 1140
   tcagtcctcg atggcactga gttcgcctac ggcaccagct ctaacctgcc ctctgctgtc 1200
   taccgcaaga gcggcaccgt tgattcgctc gatgagatcc cccctcagaa caacaacgtc 1260
   ccccctcgtc agggtttctc acatcgtctg agccatgtct cgatgttccg cagcggtttc 1320
   agcaactctt cggtctcgat catccgcgcc cctatgttct cttggattca tcgctcggcc 1380
   gagttcaaca acatcatcgc ctctgatagc atcactcaga tccccgccgt caagggcaac 1440
   ttcctgttca acggctcagt gatcagcggt cctggcttca ctggtggcga tttggtccgc 1500
   ctgaactcgt cgggcaacaa catccagaac cgtggttaca tcgaggtccc catccatttc 1560
   cctagcacct cgacccgtta ccgcgtgagg gtcaggtacg cttcggttac ccctatccat 1620
   ttgaacgtta actggggcaa ctcatcgatc ttctctaaca ccgtccccgc caccgccacc 1680
   tcgctggata acctccagag ctctgatttc ggctacttcg agagcgccaa cgccttcacc 1740
   tccagcctgg gcaacatcgt tggcgtgagg aacttctcag gcaccgctgg tgtcatcatc 1800
   gaccgcttcg agttcatccc cgtcaccgct accctggagg ctgagtacaa cctggagagg 1860
   gctcagaagg ctgtgaacgc tctcttcacc agcactaacc agctcggtct taagaccaac 1920
   gttaccgatt accatatcga ccaggtttca aacctcgtca cctacctcag cgatgagttc 1980
   tgcctggatg agaagcgcga gctgagcgag aaggtcaagc atgccaagcg cctgtcggat 2040
   gagcgcaacc tgctccagga tagcaacttc aaggatatca accgtcagcc cgagaggggc 2100
   tggggcggtt caaccggtat caccatccag ggtggcgatg atgtcttcaa ggagaactac 2160
   gtcaccctga gcggcacctt cgatgagtgc taccctacct acttgtacca gaagatcgac 2220
   gagtctaagc tcaaggcctt cactcgctac cagttgcgcg gttacatcga ggattcacag 2280
   gatctggaaa tctacctgat ccgctacaac gctaagcatg agactgttaa cgtccctggc 2340
   accggttcgc tctggcctct cagcgctcag tctcccatcg gcaagtgcgg cgagcctaac 2400
   cgttgcgccc ctcatttgga gtggaaccct gatctggatt gctcatgccg cgatggcgag 2460
   aagtgcgccc atcatagcca tcatttctca ttggatatcg atgtcggttg caccgatctg 2520
   aacgaggatc tcggtgtctg ggtcatcttc aagatcaaga cccaggatgg tcatgcccgc 2580
   ctcggcaact tggagttcct ggaggagaag cctctggtcg gcgaggcctt ggctagggtg 2640
   aagcgcgccg agaagaagtg gcgcgataag cgcgagaagc tggagtggga gaccaacatc 2700
   gtctacaagg aggccaagga gtcagtcgat gccctgttcg tgaacagcca gtacgatcag 2760
   ctccaggccg ataccaacat cgccatgatc catgccgccg ataagcgcgt tcatagcatc 2820
   cgcgaggcct acctccctga gctctcagtg atccctggtg ttaacgccgc tatcttcgag 2880
   gagctggagg gccgcatctt cactgccttc agcctgtacg atgctcgtaa cgtgatcaag 2940
   aacggtgatt tcaacaacgg tttgtcatgc tggaacgtga agggccatgt cgatgtcgag 3000
   gagcagaaca accagcgcag cgttctggtt gtccctgagt gggaggctga ggtttcacag 3060
   gaggtccgcg tctgccctgg ccgtggttac atcctgaggg tcaccgctta caaggagggt 3120
   tacggcgagg gttgcgttac tatccatgag atcgagaaca acaccgatga gctcaagttc 3180
   tcaaactgcg tcgaggagga aatctacccc aacaacactg tgacctgcaa cgattacact 3240
   gttaaccagg aggagtacgg cggcgcctac accagccgta accgtggcta caacgaggct 3300
   ccctctgtcc ccgccgatta cgcctcagtc tacgaggaga agtcgtacac cgatggccgc 3360
   cgcgagaacc cctgcgagtt caacaggggt taccgcgatt acacccccttgcccgtcggc 3420
   tacgtgacta aggagctgga gtacttcccc gagactgata aggtctggat cgagatcggc 3480
   gagactgagg gcaccttcat cgttgattca gtcgagctgc tgctcatgga ggagtga 3537
<210> 2
   <211> 3537
   <212> DNA
   <213> Artificial Sequence
<220>
   <223> synthetic nucleotide sequence encoding Cry1Ac (NalCry1Ac)
<400> 2
   atggataaca accccaacat caacgagtgc atcccctaca actgcctctc aaaccccgag 60
   gtcgaggtcc tcggcggtga gcgtatcgag accggttaca cccctatcga tatcagcctg 120
   agcctgaccc agttcctcct ctcggagttc gtccccggtg ctggtttcgt tctgggtttg 180
   gtcgatatca tctggggcat cttcggcccc tcgcagtggg atgctttcct ggtccagatc 240
   gagcagttga tcaaccagcg catcgaggag ttcgccagga accaggctat ctcacgtctg 300
   gagggtttgt cgaacctcta ccaaatctac gccgagagct tccgcgagtg ggaggctgat 360
   cctactaacc ccgctttgcg tgaggagatg cgcatccagt tcaacgatat gaactcggcc 420
   ctgaccaccg ccatccccct cttcgctgtc cagaactacc aggtcccctt gctctcagtc 480
   tacgtccagg ctgctaacct ccatctgagc gtcctgaggg atgtctctgt cttcggccag 540
   aggtggggtt tcgatgctgc tactatcaac agccgctaca acgatctcac ccgcctgatc 600
   ggcaactaca ccgattacgc cgtccgttgg tacaacaccg gcctggagcg tgtttggggt 660
   cctgattcac gtgattgggt ccgttacaac cagttccgca gggagctcac cttgaccgtc 720
   ttggatatcg tcgccctctt ccccaactac gatagccgcc gttaccccat ccgtaccgtt 780
   agccagctca ctagggaaat ctacaccaac cccgtcctgg agaacttcga tggctcgttc 840
   cgtggttcag ctcagggtat cgagaggagc atccgttcgc ctcatctgat ggatatcctg 900
   aactcgatca ccatctacac cgatgcccat cgcggctact actactggtc gggccatcag 960
   atcatggcct cgcccgtcgg cttctcaggt cctgagttca ccttcccctt gtacggcacc 1020
   atgggtaacg ccgctcctca gcagaggatc gttgctcagt tgggtcaggg tgtttacagg 1080
   accctgagct ctaccctcta caggaggcct ttcaacatcg gcatcaacaa ccagcagctg 1140
   tcagtcctcg atggcactga gttcgcctac ggcaccagct ctaacctgcc ctctgctgtc 1200
   taccgcaaga gcggcaccgt tgattcgctc gatgagatcc cccctcagaa caacaacgtc 1260
   ccccctcgtc agggtttctc acatcgtctg agccatgtct cgatgttccg cagcggtttc 1320
   agcaactctt cggtctcgat catccgcgcc cctatgttct cttggattca tcgctcggcc 1380
   gagttcaaca acatcatcgc ctctgatagc atcactcaga tccccgccgt caagggcaac 1440
   ttcctgttca acggctcagt gatcagcggt cctggcttca ctggtggcga tttggtccgc 1500
   ctgaactcgt cgggcaacaa catccagaac cgtggttaca tcgaggtccc catccatttc 1560
   cctagcacct cgacccgtta ccgcgtgagg gtcaggtacg cttcggttac ccctatccat 1620
   ttgaacgtta actggggcaa ctcatcgatc ttctctaaca ccgtccccgc caccgccacc 1680
   tcgctggata acctccagag ctctgatttc ggctacttcg agagcgccaa cgccttcacc 1740
   tccagcctgg gcaacatcgt tggcgtgagg aacttctcag gcaccgctgg tgtcatcatc 1800
   gaccgcttcg agttcatccc cgtcaccgct accctggagg ctgagtacaa cctggagagg 1860
   gctcagaagg ctgtgaacgc tctcttcacc agcactaacc agctcggtct caagaccaac 1920
   gttaccgatt accatatcga ccaggtttca aacctcgtca cctacctcag cgatgagttc 1980
   tgcctggatg agaagcgcga gctgagcgag aaggtcaagc atgccaagcg cctgtcggat 2040
   gagcgcaacc tgctccagga tagcaacttc aaggatatca accgtcagcc cgagaggggc 2100
   tggggcggtt caaccggtat caccatccag ggtggcgatg atgtcttcaa ggagaactac 2160
   gtcaccctga gcggcacctt cgatgagtgc taccctacct acttgtacca gaagatcgac 2220
   gagtctaagc tcaaggcctt cactcgctac cagttgcgcg gttacatcga ggattcacag 2280
   gatctggaaa tctacctgat ccgctacaac gctaagcatg agactgttaa cgtccctggc 2340
   accggttcgc tctggcctct cagcgctcag tctcccatcg gcaagtgcgg cgagcctaac 2400
   cgttgcgccc ctcatttgga gtggaaccct gatctggatt gctcatgccg cgatggcgag 2460
   aagtgcgccc atcatagcca tcatttctca ttggatatcg atgtcggttg caccgatctg 2520
   aacgaggatc tcggtgtctg ggtcatcttc aagatcaaga cccaggatgg tcatgcccgc 2580
   ctcggcaact tggagttcct ggaggagaag cctctggtcg gcgaggcctt ggctagggtg 2640
   aagcgcgccg agaagaagtg gcgcgataag cgcgagaagc tggagtggga gaccaacatc 2700
   gtctacaagg aggccaagga gtcagtcgat gccctgttcg tgaacagcca gtacgatcag 2760
   ctccaggccg ataccaacat cgccatgatc catgccgccg ataagcgcgt tcatagcatc 2820
   cgcgaggcct acctccctga gctctcagtg atccctggtg ttaacgccgc tatcttcgag 2880
   gagctggagg gccgcatctt cactgccttc agcctgtacg atgctcgtaa cgtgatcaag 2940
   aacggtgatt tcaacaacgg tttgtcatgc tggaacgtga agggccatgt cgatgtcgag 3000
   gagcagaaca accagcgcag cgttctggtt gtccctgagt gggaggctga ggtttcacag 3060
   gaggtccgcg tctgccctgg ccgtggttac atcctgaggg tcaccgctta caaggagggt 3120
   tacggcgagg gttgcgttac tatccatgag atcgagaaca acaccgatga gctcaagttc 3180
   tcaaactgcg tcgaggagga aatctacccc aacaacactg tgacctgcaa cgattacact 3240
   gttaaccagg aggagtacgg cggcgcctac accagccgta accgtggcta caacgaggct 3300
   ccctctgtcc ccgccgatta cgcctcagtc tacgaggaga agtcgtacac cgatggccgc 3360
   cgcgagaacc cctgcgagtt caacaggggt taccgcgatt acaccccctt gcccgtcggc 3420
   tacgtgacta aggagctgga gtacttcccc gagactgata aggtctggat cgagatcggc 3480
   gagactgagg gcaccttcat cgttgattca gtcgagctgc tgctcatgga ggagtga    3537
<210> 3
   <211> 3537
   <212> DNA
   <213> Artificial Sequence
<220>
   <223> synthetic nucleotide sequence encoding Cry1Ac (synCry1AcB)
<400> 3
   atggacaaca accccaacat caatgaatgc atcccctaca actgcttgag caacccggag 60
   gtggaggtgc tgggaggaga aagaattgaa actggctaca cgcccatcga catcagcttg 120
   agcttgacac aatttcttct ttcagaattt gttcctggcg ccggcttcgt gctggggctg 180
   gtggacatca tctggggcat cttcggccca agccaatggg atgccttcct ggtgcaaatt 240
   gagcagctca tcaaccagag gattgaagaa tttgcaagaa atcaagccat ctcaaggctg 300
   gaagggctga gcaacctcta ccagatctac gccgagagct tcagagaatg ggaagctgat 360
   ccaacaaatc ctgctcttcg agaagagatg aggattcagt tcaacgacat gaactcggcg 420
   ctcaccaccg ccatcccgct cttcgccgtc cagaactacc aagttcctct tctttcagtt 480
   tatgttcaag ctgccaacct ccacctctcc gtgctgagag atgtttctgt ttttggacaa 540
   agatggggct tcgacgccgc caccatcaac agcagataca atgatttgac aaggctcatc 600
   ggcaactaca ccgactacgc cgtccgctgg tacaacaccg gcctggagag ggtgtgggga 660
   ccagattcaa gagattgggt gagatacaac cagttcagaa gagagctcac cttgacggtg 720
   ctggacatcg tggcgctctt ccccaactat gattcaagaa gatatcccat caggacggtg 780
   agccagctga caagggagat ctacaccaac ccggtgctgg agaactttga tggcagcttc 840
   cgcggcagcg ctcaaggaat tgaaagaagc atcagaagcc ctcatctgat ggacatcctc 900
   aacagcatca ccatctacac tgatgctcac cgcggctact actactggag cggccaccag 960
   atcatggcat cacctgttgg cttctctgga cctgagttca ccttcccgct ctatggaaca 1020
   atgggcaatg ctgctcctca acaaagaatt gtggcgcagc tgggccaagg agtctacagg 1080
   acgctgagca gcaccttgta ccggcggccc ttcaacatcg gcatcaacaa ccagcagctc 1140
   tccgtgctgg atggaactga atttgcttat ggaacatcaa gcaacctgcc aagcgccgtc 1200
   tacaggaaga gcggcaccgt ggacagcttg gatgagatcc cgccgcagaa caacaatgtg 1260
   ccgccgcgcc aaggcttcag ccaccgcctc agccatgtga gcatgttcag aagcggcttc 1320
   agcaacagct ccgtcagcat catcagggcg ccgatgttca gctggattca tcgctcagca 1380
   gagttcaaca acatcattgc ttctgacagc atcacccaga tcccggcggt gaagggcaac 1440
   ttcctcttca atggaagcgt catctctgga cctggcttca ctggaggaga tctggtgagg 1500
   ctcaacagca gcggcaacaa catccagaac agaggctaca ttgaggtgcc catccacttc 1560
   ccatcaacat caacaagata cagggtgagg gtgagatatg cttctgtgac gcccatccac 1620
   ctcaatgtca actggggcaa cagcagcatc ttcagcaaca ccgtgccggc gacggcgacg 1680
   agcttggaca accttcaaag ctcagatttt ggatattttg agagcgccaa cgccttcacc 1740
   tcctcgctgg gcaacattgt tggtgtgagg aacttcagcg gcaccgccgg cgtcatcatc 1800
   gacagatttg agttcatccc ggtgacagca acattggagg cggagtacaa cctagaaaga 1860
   gctcagaagg ccgtcaacgc gctcttcacc tccaccaacc agctgggcct caagacaaat 1920
   gtcaccgact accacattga tcaagtgagc aacctggtga cctacctctc tgatgagttc 1980
   tgcttggatg agaagaggga gctctccgag aaggtgaagc atgccaagag gctctctgat 2040
   gaaaggaacc tgctgcaaga ttcaaacttc aaggacatca acaggcagcc agaaagagga 2100
   tggggaggaa gcaccggcat caccatccaa ggaggagatg atgtcttcaa ggagaactac 2160
   gtcaccttga gcggcacctt tgatgaatgc taccccacct acctctacca gaagattgat 2220
   gaaagcaagc taaaggcctt cacaagatac cagctccgcg gctacattga agattctcaa 2280
   gatctggaga tctacctcat cagatacaac gccaagcatg agacggtgaa tgttcctgga 2340
   actggaagcc tctggccgct ctcagctcaa agccccatcg gcaaatgtgg agagcccaac 2400
   cgctgcgcgc cgcacctgga gtggaatcca gatctggatt gcagctgccg agatggagaa 2460
   aaatgtgctc atcacagcca ccacttctca ttggacattg atgttggctg caccgacctc 2520
   aatgaagatc ttggagtttg ggtgatcttc aagatcaaga ctcaagatgg ccatgcaagg 2580
   ctgggcaacc tggagttcct ggaggagaag ccgctggtgg gagaagctct agcaagggtg 2640
   aagagagctg agaagaagtg gagggacaag agggagaagc tggagtggga gaccaacatc 2700
   gtctacaagg aggccaagga gagcgtggat gctctcttcg tcaacagcca atatgatcaa 2760
   cttcaagctg acaccaacat cgccatgatc cacgccgccg acaagagggt gcacagcatc 2820
   agagaagcat atcttccaga gctctcagtg atccccggcg tcaacgccgc catcttcgag 2880
   gagctggaag gaaggatctt caccgccttc agcctctatg atgcaagaaa tgtcatcaag 2940
   aatggagact tcaacaatgg gctgagctgc tggaatgtga agggccatgt tgatgtggag 3000
   gagcagaaca accaaagatc agtgctggtg gtgccagaat gggaagcaga agtttctcaa 3060
   gaagttcgag tttgccccgg ccgcggctac atcctccgcg tcaccgccta caaggaagga 3120
   tatggagaag gctgcgtcac catccatgag attgagaaca acaccgacga gctgaagttc 3180
   tcaaattgtg tggaggagga gatctacccc aacaacaccg tcacctgcaa tgactacacc 3240
   gtgaaccaag aagaatatgg cggcgcctac acctcaagga acagaggcta caatgaagct 3300
   ccttctgttc ctgctgatta tgcttctgtc tacgaggaga agagctacac tgatggaaga 3360
   agagaaaatc catgtgagtt caacagaggc tacagggact acacgccgct acctgttgga 3420
   tatgtgacca aggagctgga gtacttccca gaaactgaca aggtgtggat tgagattgga 3480
   gaaacagaag gaaccttcat cgtggacagc gtggagctgc tgctgatgga ggagtag 3537
<210> 4
   <211> 3537
   <212> DNA
   <213> Artificial Sequence
<220>
   <223> synthetic nucleotide sequence encoding Cry1Ac (synCry1AcC)
<400> 4
   atggacaaca accccaacat caatgaatgc atcccctaca actgcttgag caacccggag 60
   gtggaggtgc tgggaggaga aagaattgaa actggctaca cgcccatcga catcagcttg 120
   agcttgacac aatttcttct ttcagaattt gttcctggag ctggcttcgt gctgggcttg 180
   gtggacatca tctggggcat ctttggacca agccaatggg atgccttctt ggtgcaaatt 240
   gagcagctca tcaaccaaag aattgaagaa tttgcaagaa atcaagccat ctcaaggctg 300
   gaaggattga gcaacctcta ccagatctat gctgagagct tcagagaatg ggaagctgat 360
   ccaacaaatc ctgctcttcg agaagaaatg aggattcaat tcaatgacat gaactcggcg 420
   ctcaccaccg ccatcccgct cttcgccgtc cagaactacc aagttcctct tctttcagtt 480
   tatgttcaag ctgccaacct ccacctctcc gtgctgagag atgtttctgt ttttggacaa 540
   agatggggct tcgacgccgc caccatcaac tcaagataca atgatttgac aaggctcatc 600
   ggcaactaca ccgactacgc cgtcagatgg tacaacaccg gcctggagag agtttgggga 660
   ccagattcaa gagattgggt gagatacaac cagttcagaa gagagctgac attgacagtg 720
   ctggacattg tggcgctctt cccaaattat gattcaagaa gatatcccat caggacggtg 780
   agccagctga caagagagat ctacaccaac ccggtgctgg agaattttga tggcagcttc 840
   cgcggctctg ctcaaggaat tgaaagaagc atcagatctc ctcatttgat ggacatcctc 900
   aacagcatca ccatctacac tgatgctcat cgaggctact actactggag cggccaccag 960
   atcatggcat cacctgttgg cttctctgga cctgagttca ccttcccgct ctatggaaca 1020
   atgggaaatg ctgctcctca acaaagaatt gtggcgcagc tgggccaagg agtctacagg 1080
   acattgagca gcaccttgta ccggcggcca ttcaacatcg gcatcaacaa ccagcagctc 1140
   tccgtgctgg atggaactga atttgcttat ggaacatcaa gcaaccttcc aagcgccgtc 1200
   tacaggaaga gcggcaccgt ggacagcttg gatgagatcc cgccgcagaa caacaatgtg 1260
   ccgccgcgcc aaggcttcag ccaccgcctc agccatgtga gcatgttcag aagtggcttc 1320
   agcaacagct ccgtcagcat catcagggcg ccaatgttca gctggattca tcgctcagca 1380
   gagttcaaca acatcattgc ttctgacagc atcacccaga tcccggcggt gaagggcaac 1440
   ttcctcttca atggaagcgt catctctgga cctggcttca ctggaggaga tttggtgagg 1500
   ctcaacagca gcggcaacaa catccagaac agaggctaca ttgaagttcc catccacttc 1560
   ccatcaacat caacaagata cagggtgagg gtgagatatg cttctgtgac gcccatccac 1620
   ctcaatgtca actggggcaa cagcagcatc ttcagcaaca ccgtgccggc gacggcgacg 1680
   agcttggaca accttcaaag ctcagatttt ggatattttg aaagcgccaa cgccttcacc 1740
   agcagcttgg gcaacattgt tggtgtgagg aacttcagcg gcaccgccgg cgtcatcatc 1800
   gacagatttg agttcatccc ggtgacagca acattggaag ctgagtacaa cctagaaaga 1860
   gctcagaagg ccgtcaatgc tctcttcacc tccaccaacc agctgggctt gaagacaaat 1920
   gtcaccgact accacattga tcaagtttca aatttggtga cctacctctc tgatgagttc 1980
   tgcttggatg agaagaggga gctctcagag aaggtgaagc atgccaagag gctttctgat 2040
   gaaagaaatt tgctgcaaga ttcaaacttc aaggacatca acaggcaacc agaaagagga 2100
   tggggaggaa gcaccggcat caccatccaa ggaggagatg atgtcttcaa ggagaactat 2160
   gtcaccttga gcggcacctt tgatgaatgc tatccaacct acctctacca gaagattgat 2220
   gaaagcaagc taaaggcctt cacaagatat cagctgagag gctacattga agattctcaa 2280
   gatttggaga tctacctcat cagatacaat gccaagcatg aaacagtgaa tgttcctgga 2340
   actggaagcc tctggccgct ctcagctcaa agccccattg gaaaatgtgg agaaccaaac 2400
   agatgcgcgc cgcacctgga atggaatcca gatcttgatt gcagctgccg agatggagaa 2460
   aaatgtgctc atcacagcca tcacttctca ttggacattg atgttggatg caccgacctc 2520
   aatgaagatc ttggagtttg ggtgatcttc aagatcaaga ctcaagatgg acatgcaagg 2580
   ctgggcaacc tggagttctt ggaggagaag ccgctggtgg gagaagctct agcaagggtg 2640
   aagagagctg agaagaaatg gagagacaag agggagaagc tggaatggga gaccaacatc 2700
   gtctacaagg aggccaagga gagcgtggat gctctcttcg tcaacagcca atatgatcaa 2760
   cttcaagctg acaccaacat cgccatgatc cacgccgccg acaagagggt gcacagcatc 2820
   agagaagcat atcttccaga gctctcagtg attcctggcg tcaacgccgc catcttcgag 2880
   gagctggaag gaaggatctt caccgccttc agcctctatg atgcaagaaa tgtcatcaag 2940
   aatggagatt tcaacaatgg cttgagctgc tggaatgtga agggccatgt tgatgtggag 3000
   gagcagaaca accaaagatc agtgctggtg gtgccagaat gggaagcaga agtttctcaa 3060
   gaagttcgag tttgtcctgg aagaggctac atcctccgcg tcaccgccta caaagaagga 3120
   tatggagaag gatgtgtcac catccatgaa attgagaaca acactgatga gctgaagttc 3180
   tcaaattgtg tggaggagga gatctacccc aacaacaccg tcacctgcaa tgactacacc 3240
   gtgaatcaag aagaatatgg cggcgcctac acctcaagaa acagaggcta caatgaagct 3300
   ccttctgttc ctgctgatta tgcttctgtt tatgaggaga agagctacac tgatggaaga 3360
   agagaaaatc catgtgagtt caacagaggc tacagagact acacgccgct acctgttgga 3420
   tatgtgacaa aggagctgga gtacttccca gaaactgaca aggtgtggat tgaaattgga 3480
   gaaacagaag gaaccttcat cgtggattct gtggagctgc tgctgatgga agaataa    3537
<210> 5
   <211> 3537
   <212> DNA
   <213> Artificial Sequence
<220>
   <223> synthetic nucleotide sequence encoding Cry1Ac (optCry1Acv02)
<400> 5
   atggacaaca accccaacat caatgaatgc atcccctaca actgcttgag caacccagag 60
   gtggaggtgc tgggaggaga aaggattgaa actggctaca cccccatcga catctccctc 120
   tccctcaccc agttcctcct ctcagaattt gttcctggag ctggcttcgt gctggggctg 180
   gtggacatca tctggggcat cttcggccct tctcaatggg atgccttcct cgtccagatc 240
   gagcagctga tcaaccagag gattgaagaa tttgcaagga accaggccat ctcaaggctg 300
   gaaggcctct ccaacctcta ccagatttat gctgagagct tcagagaatg ggaagcagat 360
   ccaacaaatc ctgctctgag ggaggagatg aggattcagt tcaatgacat gaactcagct 420
   ctcaccaccg ccatccctct cttcgccgtc cagaactacc aggtgccgct gctctccgtc 480
   tatgttcaag ctgccaacct ccacctctcc gtgctgagag atgtttcagt ttttggccaa 540
   agatggggct ttgatgctgc caccatcaac agcagataca atgatctgac aaggctcatc 600
   ggcaactaca cagattatgc tgtcagatgg tacaacaccg gcctggagcg cgtctggggg 660
   ccagattcaa gagattgggt gagatacaac cagttcagaa gggagctcac cttgacggtg 720
   ctggacatcg tcgccctctt ccccaactat gattcaagaa gatatcccat caggaccgtc 780
   agccagctga caagggagat ctacaccaac cccgtgctgg agaactttga tggcagcttc 840
   agaggatcag ctcaaggaat tgaaagaagc atcagatctc ctcatctgat ggacatcctc 900
   aacagcatca ccatctacac agatgctcac cgcggctact actactggag cggccaccag 960
   atcatggctt ctcctgttgg cttctcagga cctgagttca ccttccctct ctatggcacc 1020
   atgggcaacg ccgcgccgca gcagaggatc gtcgcccagc tgggccaagg agtctacagg 1080
   accttgagca gcaccctcta caggaggccc ttcaacatcg gcatcaacaa ccagcagctc 1140
   tccgtgctgg atggaactga atttgcatat ggaacaagca gcaaccttcc ttcagctgtc 1200
   tacaggaaga gcggcaccgt ggacagcttg gatgaaattc ctcctcaaaa caacaatgtg 1260
   ccgccgcgcc aaggcttcag ccaccgcctc agccatgtga gcatgttcag aagcggcttc 1320
   agcaacagca gcgtcagcat catcagggcg ccgatgttca gctggattca ccgctcagca 1380
   gagttcaaca acatcattgc ttctgacagc atcacccaga tccccgccgt caagggcaac 1440
   ttcctcttca atggcagcgt catctccggc cctggcttca ctggaggaga tctggtgagg 1500
   ctcaacagca gcggcaacaa catccagaac agaggctaca ttgaggtgcc catccacttc 1560
   ccctccacct caacaagata cagggtgagg gtgagatatg cttctgtcac ccccatccac 1620
   ctcaatgtca actggggcaa cagcagcatc ttcagcaaca ctgttccagc aacagcaaca 1680
   agcctggaca accttcaaag ctcagatttt ggatattttg aatcagcaaa tgccttcacc 1740
   agcagcttgg gcaacattgt tggagtgagg aacttctccg gcaccgccgg cgtcatcatc 1800
   gacagatttg agttcatccc cgtcaccgcc accttggaag cagagtacaa cctggagaga 1860
   gctcagaagg ccgtcaatgc tctcttcacc tcaacaaacc agctgggcct caagacaaat 1920
   gtcaccgact accacattga tcaagtcagc aacctggtga catatctctc tgatgagttc 1980
   tgcttggatg agaagaggga gctctcagag aaggtgaagc atgccaagag gctctctgat 2040
   gaaaggaacc tcctccagga cagcaacttc aaggacatca acaggcagcc agaaagagga 2100
   tggggaggaa gcaccggcat caccatccaa ggaggagatg atgttttcaa ggagaactat 2160
   gtcaccctct ccggcacctt tgatgaatgc taccccacct acctctacca gaagattgat 2220
   gaatcaaagc tgaaggcctt cacaagatat cagctccgcg gctacatcga ggacagccaa 2280
   gatctggaga tctacctcat cagatacaat gccaagcatg aaactgtcaa tgttcctgga 2340
   actggaagcc tctggccgct gagcgctcaa agccccattg gaaaatgtgg agaaccaaac 2400
   agatgtgctc ctcatctgga atggaatcca gatctggact gctcatgccg agatggagaa 2460
   aaatgtgctc accacagcca ccacttctcc ttggacattg atgttggctg caccgacctc 2520
   aatgaagatc ttggagtttg ggtgatcttc aagatcaaga ctcaagatgg ccatgcaagg 2580
   ctgggcaacc tggagttcct ggaggagaag ccgctggtgg gagaagctct agcaagggtg 2640
   aagagagctg agaagaaatg gagggacaag agggagaagc tggaatggga aacaaacatc 2700
   gtctacaagg aggccaagga gagcgtggat gctctcttcg tcaacagcca atatgatcag 2760
   ctgcaagctg acaccaacat tgccatgatc catgctgctg acaagagggt gcattcaatc 2820
   agagaagcat atcttccaga gctctccgtc atccccggcg tcaatgctgc catcttcgag 2880
   gagctggaag gaaggatctt caccgccttc tccctctatg atgcaagaaa tgtcatcaag 2940
   aatggagatt tcaacaatgg cctgagctgc tggaatgtca agggccatgt tgatgtggag 3000
   gagcagaaca accaaagatc agtgctggtg gtgccagaat gggaagcaga agtttctcaa 3060
   gaagtccgcg tctgccctgg aagaggctac atcctccgcg tcaccgccta caaggaagga 3120
   tatggagaag gctgcgtcac catccatgag attgagaaca acacagatga gctgaagttc 3180
   tcaaactgcg tggaggagga gatctacccc aacaacaccg tcacatgcaa tgactacacc 3240
   gtgaaccaag aagaatatgg cggcgcctac acctcaagga acagaggcta caatgaagct 3300
   ccttctgttc ctgctgatta tgcttctgtt tatgaggaga agagctacac agatggaaga 3360
   agggagaacc cctgcgagtt caacagaggc tacagggact acacgccgct acctgttgga 3420
   tatgtgacca aggagctgga gtacttccca gaaacagaca aggtctggat tgagattgga 3480
   gaaacagaag gaaccttcat cgtggacagc gtggagctgc tgctgatgga ggagtaa    3537
<210> 6
   <211> 1178
   <212> PRT
   <213> Bacillus thuringiensis
<400> 6
   Met Asp Asn Asn Pro Asn Ile Asn Glu Cys Ile Pro Tyr Asn Cys Leu
   1               5                   10                  15
   Ser Asn Pro Glu Val Glu Val Leu Gly Gly Glu Arg Ile Glu Thr Gly
               20                  25                  30
   Tyr Thr Pro Ile Asp Ile Ser Leu Ser Leu Thr Gln Phe Leu Leu Ser
           35                  40                  45
   Glu Phe Val Pro Gly Ala Gly Phe Val Leu Gly Leu Val Asp Ile Ile
       50                  55                  60
   Trp Gly Ile Phe Gly Pro Ser Gln Trp Asp Ala Phe Leu Val Gln Ile
   65                  70                  75                  80
   Glu Gln Leu Ile Asn Gln Arg Ile Glu Glu Phe Ala Arg Asn Gln Ala
                   85                  90                  95
   Ile Ser Arg Leu Glu Gly Leu Ser Asn Leu Tyr Gln Ile Tyr Ala Glu
               100                 105                 110
   Ser Phe Arg Glu Trp Glu Ala Asp Pro Thr Asn Pro Ala Leu Arg Glu
           115                 120                 125
   Glu Met Arg Ile Gln Phe Asn Asp Met Asn Ser Ala Leu Thr Thr Ala
       130                 135                 140
   Ile Pro Leu Phe Ala Val Gln Asn Tyr Gln Val Pro Leu Leu Ser Val
   145                 150                 155                 160
   Tyr Val Gln Ala Ala Asn Leu His Leu Ser Val Leu Arg Asp Val Ser
                   165                 170                 175
   Val Phe Gly Gln Arg Trp Gly Phe Asp Ala Ala Thr Ile Asn Ser Arg
               180                 185                 190
   Tyr Asn Asp Leu Thr Arg Leu Ile Gly Asn Tyr Thr Asp Tyr Ala Val
           195                 200                 205
   Arg Trp Tyr Asn Thr Gly Leu Glu Arg Val Trp Gly Pro Asp Ser Arg
       210                 215                 220
   Asp Trp Val Arg Tyr Asn Gln Phe Arg Arg Glu Leu Thr Leu Thr Val
   225                 230                 235                 240
   Leu Asp Ile Val Ala Leu Phe Pro Asn Tyr Asp Ser Arg Arg Tyr Pro
                   245                 250                 255
   Ile Arg Thr Val Ser Gln Leu Thr Arg Glu Ile Tyr Thr Asn Pro Val
               260                 265                 270
   Leu Glu Asn Phe Asp Gly Ser Phe Arg Gly Ser Ala Gln Gly Ile Glu
           275                 280                 285
   Arg Ser Ile Arg Ser Pro His Leu Met Asp Ile Leu Asn Ser Ile Thr
       290                 295                 300
   Ile Tyr Thr Asp Ala His Arg Gly Tyr Tyr Tyr Trp Ser Gly His Gln
   305                 310                 315                 320
   Ile Met Ala Ser Pro Val Gly Phe Ser Gly Pro Glu Phe Thr Phe Pro
                   325                 330                 335
   Leu Tyr Gly Thr Met Gly Asn Ala Ala Pro Gln Gln Arg Ile Val Ala
               340                 345                 350
   Gln Leu Gly Gln Gly Val Tyr Arg Thr Leu Ser Ser Thr Leu Tyr Arg
           355                 360                 365
   Arg Pro Phe Asn Ile Gly Ile Asn Asn Gln Gln Leu Ser Val Leu Asp
       370                 375                 380
   Gly Thr Glu Phe Ala Tyr Gly Thr Ser Ser Asn Leu Pro Ser Ala Val
   385                 390                 395                 400
   Tyr Arg Lys Ser Gly Thr Val Asp Ser Leu Asp Glu Ile Pro Pro Gln
                   405                 410                 415
   Asn Asn Asn Val Pro Pro Arg Gln Gly Phe Ser His Arg Leu Ser His
               420                 425                 430
   Val Ser Met Phe Arg Ser Gly Phe Ser Asn Ser Ser Val Ser Ile Ile
           435                 440                 445
   Arg Ala Pro Met Phe Ser Trp Ile His Arg Ser Ala Glu Phe Asn Asn
       450                 455                 460
   Ile Ile Ala Ser Asp Ser Ile Thr Gln Ile Pro Ala Val Lys Gly Asn
   465                 470                 475                 480
   Phe Leu Phe Asn Gly Ser Val Ile Ser Gly Pro Gly Phe Thr Gly Gly
                   485                 490                 495
   Asp Leu Val Arg Leu Asn Ser Ser Gly Asn Asn Ile Gln Asn Arg Gly
               500                 505                 510
   Tyr Ile Glu Val Pro Ile His Phe Pro Ser Thr Ser Thr Arg Tyr Arg
           515                 520                 525
   Val Arg Val Arg Tyr Ala Ser Val Thr Pro Ile His Leu Asn Val Asn
       530                 535                 540
   Trp Gly Asn Ser Ser Ile Phe Ser Asn Thr Val Pro Ala Thr Ala Thr
   545                 550                 555                 560
   Ser Leu Asp Asn Leu Gln Ser Ser Asp Phe Gly Tyr Phe Glu Ser Ala
                   565                 570                 575
   Asn Ala Phe Thr Ser Ser Leu Gly Asn Ile Val Gly Val Arg Asn Phe
               580                 585                 590
   Ser Gly Thr Ala Gly Val Ile Ile Asp Arg Phe Glu Phe Ile Pro Val
           595                 600                 605
   Thr Ala Thr Leu Glu Ala Glu Tyr Asn Leu Glu Arg Ala Gln Lys Ala
       610                 615                 620
   Val Asn Ala Leu Phe Thr Ser Thr Asn Gln Leu Gly Leu Lys Thr Asn
   625                 630                 635                 640
   Val Thr Asp Tyr His Ile Asp Gln Val Ser Asn Leu Val Thr Tyr Leu
                   645                 650                 655
   Ser Asp Glu Phe Cys Leu Asp Glu Lys Arg Glu Leu Ser Glu Lys Val
               660                 665                 670
   Lys His Ala Lys Arg Leu Ser Asp Glu Arg Asn Leu Leu Gln Asp Ser
           675                 680                 685
   Asn Phe Lys Asp Ile Asn Arg Gln Pro Glu Arg Gly Trp Gly Gly Ser
       690                 695                 700
   Thr Gly Ile Thr Ile Gln Gly Gly Asp Asp Val Phe Lys Glu Asn Tyr
   705                 710                 715                 720
   Val Thr Leu Ser Gly Thr Phe Asp Glu Cys Tyr Pro Thr Tyr Leu Tyr
                   725                 730                 735
   Gln Lys Ile Asp Glu Ser Lys Leu Lys Ala Phe Thr Arg Tyr Gln Leu
               740                 745                 750
   Arg Gly Tyr Ile Glu Asp Ser Gln Asp Leu Glu Ile Tyr Leu Ile Arg
           755                 760                 765
   Tyr Asn Ala Lys His Glu Thr Val Asn Val Pro Gly Thr Gly Ser Leu
       770                 775                 780
   Trp Pro Leu Ser Ala Gln Ser Pro Ile Gly Lys Cys Gly Glu Pro Asn
   785                 790                 795                 800
   Arg Cys Ala Pro His Leu Glu Trp Asn Pro Asp Leu Asp Cys Ser Cys
                   805                 810                 815
   Arg Asp Gly Glu Lys Cys Ala His His Ser His His Phe Ser Leu Asp
               820                 825                 830
   Ile Asp Val Gly Cys Thr Asp Leu Asn Glu Asp Leu Gly Val Trp Val
           835                 840                 845
   Ile Phe Lys Ile Lys Thr Gln Asp Gly His Ala Arg Leu Gly Asn Leu
       850                 855                 860
   Glu Phe Leu Glu Glu Lys Pro Leu Val Gly Glu Ala Leu Ala Arg Val
   865                 870                 875                 880
   Lys Arg Ala Glu Lys Lys Trp Arg Asp Lys Arg Glu Lys Leu Glu Trp
                   885                 890                 895
   Glu Thr Asn Ile Val Tyr Lys Glu Ala Lys Glu Ser Val Asp Ala Leu
               900                 905                 910
   Phe Val Asn Ser Gln Tyr Asp Gln Leu Gln Ala Asp Thr Asn Ile Ala
           915                 920                 925
   Met Ile His Ala Ala Asp Lys Arg Val His Ser Ile Arg Glu Ala Tyr
       930                 935                 940
   Leu Pro Glu Leu Ser Val Ile Pro Gly Val Asn Ala Ala Ile Phe Glu
   945                 950                 955                 960
   Glu Leu Glu Gly Arg Ile Phe Thr Ala Phe Ser Leu Tyr Asp Ala Arg
                   965                 970                 975
   Asn Val Ile Lys Asn Gly Asp Phe Asn Asn Gly Leu Ser Cys Trp Asn
               980                 985                 990
   Val Lys Gly His Val Asp Val Glu Glu Gln Asn Asn Gln Arg Ser Val
           995                 1000                1005
   Leu Val Val Pro Glu Trp Glu Ala Glu Val Ser Gln Glu Val Arg Val
       1010                1015                1020
   Cys Pro Gly Arg Gly Tyr Ile Leu Arg Val Thr Ala Tyr Lys Glu Gly
   1025                1030                1035                1040
   Tyr Gly Glu Gly Cys Val Thr Ile His Glu Ile Glu Asn Asn Thr Asp
                   1045                1050                1055
   Glu Leu Lys Phe Ser Asn Cys Val Glu Glu Glu Ile Tyr Pro Asn Asn
               1060                1065                1070
   Thr Val Thr Cys Asn Asp Tyr Thr Val Asn Gln Glu Glu Tyr Gly Gly
           1075                1080                1085
   Ala Tyr Thr Ser Arg Asn Arg Gly Tyr Asn Glu Ala Pro Ser Val Pro
       1090                1095                1100
   Ala Asp Tyr Ala Ser Val Tyr Glu Glu Lys Ser Tyr Thr Asp Gly Arg
   1105                1110                1115                1120
   Arg Glu Asn Pro Cys Glu Phe Asn Arg Gly Tyr Arg Asp Tyr Thr Pro
                   1125                1130                1135
   Leu Pro Val Gly Tyr Val Thr Lys Glu Leu Glu Tyr Phe Pro Glu Thr
               1140                1145                1150
   Asp Lys Val Trp Ile Glu Ile Gly Glu Thr Glu Gly Thr Phe Ile Val
           1155                1160                1165
   Asp Ser Val Glu Leu Leu Leu Met Glu Glu
       1170                1175
<210> 7
   <211> 4
   <212> PRT
   <213> Artificial Sequence
<220>
   <223> endoplasmic reticulum retention sequence
<400> 7
   Lys Asp Glu Leu
    1
<210> 8
   <211> 3537
   <212> DNA
   <213> Bacillus thurigiensis
<400> 8
   atggataaca atccgaacat caatgaatgc attccttata attgtttaag taaccctgaa 60
   gtagaagtat taggtggaga aagaatagaa actggttaca ccccaatcga tatttccttg 120
   tcgctaacgc aatttctttt gagtgaattt gttcccggtg ctggatttgt gttaggacta 180
   gttgatataa tatggggaat ttttggtccc tctcaatggg acgcatttct tgtacaaatt 240
   gaacagttaa ttaaccaaag aatagaagaa ttcgctagga accaagccat ttctagatta 300
   gaaggactaa gcaatcttta tcaaatttac gcagaatctt ttagagagtg ggaagcagat 360
   cctactaatc cagcattaag agaagagatg cgtattcaat tcaatgacat gaacagtgcc 420
   cttacaaccg ctattcctct ttttgcagtt caaaattatc aagttcctct tttatcagta 480
   tatgttcaag ctgcaaattt acatttatca gttttgagag atgtttcagt gtttggacaa 540
   aggtggggat ttgatgccgc gactatcaat agtcgttata atgatttaac taggcttatt 600
   ggcaactata cagattatgc tgtacgctgg tacaatacgg gattagaacg tgtatgggga 660
   ccggattcta gagattgggt aaggtataat caatttagaa gagaattaac actaactgta 720
   ttagatatcg ttgctctgtt cccgaattat gatagtagaa gatatccaat tcgaacagtt 780
   tcccaattaa caagagaaat ttatacaaac ccagtattag aaaattttga tggtagtttt 840
   cgaggctcgg ctcagggcat agaaagaagt attaggagtc cacatttgat ggatatactt 900
   aacagtataa ccatctatac ggatgctcat aggggttatt attattggtc agggcatcaa 960
   ataatggctt ctcctgtcgg tttttcgggg ccagaattca cgtttccgct atatggaacc 1020
   atgggaaatg cagctccaca acaacgtatt gttgctcaac taggtcaggg cgtgtataga 1080
   acattatcgt ccactttata tagaagacct tttaatatag ggataaataa tcaacaacta 1140
   tctgttcttg acgggacaga atttgcttat ggaacctcct caaatttgcc atccgctgta 1200
   tacagaaaaa gcggaacggt agattcgctg gatgaaatac cgccacagaa taacaacgtg 1260
   ccacctaggc aaggatttag tcatcgatta agccatgttt caatgtttcg ttcaggcttt 1320
   agtaatagta gtgtaagtat aataagagct cctatgttct cttggataca tcgtagtgct 1380
   gaatttaata atataattgc atcggatagt attactcaaa tccctgcagt gaagggaaac 1440
   tttcttttta atggttctgt aatttcagga ccaggattta ctggtgggga cttagttaga 1500
   ttaaatagta gtggaaataa cattcagaat agagggtata ttgaagttcc aattcacttc 1560
   ccatcgacat ctaccagata tcgagttcgt gtacggtatg cttctgtaac cccgattcac 1620
   ctcaacgtta attggggtaa ttcatccatt ttttccaata cagtaccagc tacagctacg 1680
   tcattagata atctacaatc aagtgatttt ggttattttg aaagtgccaa tgcttttaca 1740
   tcttcattag gtaatatagt aggtgttaga aattttagtg ggactgcagg agtgataata 1800
   gacagatttg aatttattcc agttactgca acactcgagg ctgaatataa tctggaaaga 1860
   gcgcagaagg cggtgaatgc gctgtttacg tctacaaacc aactagggct aaaaacaaat 1920
   gtaacggatt atcatattga tcaagtgtcc aatttagtta cgtatttatc ggatgaattt 1980
   tgtctggatg aaaagcgaga attgtccgag aaagtcaaac atgcgaagcg actcagtgat 2040
   gaacgcaatt tactccaaga ttcaaatttc aaagacatta ataggcaacc agaacgtggg 2100
   tggggcggaa gtacagggat taccatccaa ggaggggatg acgtatttaa agaaaattac 2160
   gtcacactat caggtacctt tgatgagtgc tatccaacat atttgtatca aaaaatcgat 2220
   gaatcaaaat taaaagcctt tacccgttat caattaagag ggtatatcga agatagtcaa 2280
   gacttagaaa tctatttaat tcgctacaat gcaaaacatg aaacagtaaa tgtgccaggt 2340
   acgggttcct tatggccgct ttcagcccaa agtccaatcg gaaagtgtgg agagccgaat 2400
   cgatgcgcgc cacaccttga atggaatcct gacttagatt gttcgtgtag ggatggagaa 2460
   aagtgtgccc atcattcgca tcatttctcc ttagacattg atgtaggatg tacagactta 2520
   aatgaggacc taggtgtatg ggtgatcttt aagattaaga cgcaagatgg gcacgcaaga 2580
   ctagggaatc tagagtttct cgaagagaaa ccattagtag gagaagcgct agctcgtgtg 2640
   aaaagagcgg agaaaaaatg gagagacaaa cgtgaaaaat tggaatggga aacaaatatc 2700
   gtttataaag aggcaaaaga atctgtagat gctttatttg taaactctca atatgatcaa 2760
   ttacaagcgg atacgaatat tgccatgatt catgcggcag ataaacgtgt tcatagcatt 2820
   cgagaagctt atctgcctga gctgtctgtg attccgggtg tcaatgcggc tatttttgaa 2880
   gaattagaag ggcgtatttt cactgcattc tccctatatg atgcgagaaa tgtcattaaa 2940
   aatggtgatt ttaataatgg cttatcctgc tggaacgtga aagggcatgt agatgtagaa 3000
   gaacaaaaca accaacgttc ggtccttgtt gttccggaat gggaagcaga agtgtcacaa 3060
   gaagttcgtg tctgtccggg tcgtggctat atccttcgtg tcacagcgta caaggaggga 3120
   tatggagaag gttgcgtaac cattcatgag atcgagaaca atacagacga actgaagttt 3180
   agcaactgcg tagaagagga aatctatcca aataacacgg taacgtgtaa tgattatact 3240
   gtaaatcaag aagaatacgg aggtgcgtac acttctcgta atcgaggata taacgaagct 3300
   ccttccgtac cagctgatta tgcgtcagtc tatgaagaaa aatcgtatac agatggacga 3360
   agagagaatc cttgtgaatt taacagaggg tatagggatt acacgccact accagttggt 3420
   tatgtgacaa aagaattaga atacttccca gaaaccgata aggtatggat tgagattgga 3480
   gaaacggaag gaacatttat cgtggacagc gtggaattac tccttatgga ggaatag    3537